# EUROPEAN PATENT APPLICATION

(11) **EP 1 983 045 A2**
(43) Date of publication of application: **22.10.2008**
(21) Application number: 08156242.3
(22) Date of filing: 01.07.2004
(51) Int. Cl.: C12N 9/26, C12N 9/10

(54) **CGTase Variants**

(30) Priority: 01.07.2003 DK 200300994
(62) Divisional of application: 04738965.5
(71) Applicant: Novozymes A/S, 2880 Bagsvaerd (DK)
(72) Inventor: Svendsen, Allan, 2970 Hoersholm (DK); Beier, Lars, 2800 Lyngby (DK)

(57) **Abstract**

The inventors have developed a method of modifying the amino acid sequence of a CGTase to obtain variants. The variants may form linear oligosaccharides as an initial product by starch hydrolysis and a reduced amount of cyclodextrin and may be useful for anti-staling in baked products. The method is based on a comparison of three-dimensional (3D) structures of the CGTase with the structure of a maltogenic alpha-amylase where one or both models include a substrate. The invention also provides novel CGTase variants.

## Description

### FIELD OF THE INVENTION

The present invention relates to the construction of variants of cyclodextrin glucanotransferases (CGTases), in particular variants having the ability to form linear oligosaccharides.

### BACKGROUND OF THE INVENTION

Pdb files 1CDG, 1 PAM, 1 CYG and 1CIU (available at www.rcsb.org) show the amino acid sequences and three-dimensional structures of several cyclodextrin glucanotransferases (CGTases). WO 9943794 shows the amino acid sequence and three-dimensional structure of a maltogenic alpha-amylase from *Bacillus stearothermophilus,* known as Novamyl ®.

Variants of a cyclodextrin glucanotransferase (CGTase) have been described in the prior art: WO 2004026043. WO 9943793. R.J. Leemhuis: "What makes cyclodextrin glycosyltransferase a transglycosylase", University Library Groningen, 2003. H. Leemhuis et al., Journal of Biotechnology, 103 (2003), 203-212. H. Leemhuis et al., Biochemistry, 2003, 42, 7518-7526.

L. Beier et al., Protein Engineering, vol 13, no. 7, pp. 509-513, 2000 is titled "Conversion of the maltogenic α-amylase Novamyl into a CGTase".

### SUMMARY OF THE INVENTION

The inventors have developed a method of modifying the amino acid sequence of a CGTase to obtain variants. The variants may form linear oligosaccharides as an initial product by starch hydrolysis and a reduced amount of cyclodextrin and may be useful for anti-staling in baked products. The method is based on a comparison of three-dimensional (3D) structures of the CGTase with the structure of a maltogenic alpha-amylase where one or both models includes a substrate. The invention also provides novel CGTase variants.

Accordingly, the invention provides a method of producing a variant polypeptide, which method comprises:
a) providing an amino acid sequence and a three-dimensional model for a cyclodextrin glucanotransferase (CGTase) and for an amino acid sequence for a maltogenic alpha-amylase wherein one or both models includes a substrate,
b) superimposing the two three-dimensional models,
c) selecting an amino acid residue in the CGTase which:
   i) has a C-alpha atom located > 0.8 Å from the C-alpha atom of any amino acid residue in the maltogenic alpha-amylase and is located < 10 Å from an atom of a substrate,
   ii) has a C-alpha atom located < 6 Å from a non-H atom of an amino acid residue of the maltogenic alpha-amylase corresponding to residue 190-194 of SEO ID NO: 17, or
   iii) is in a subsequence (a "loop") of the CGTase wherein each residue has a C-alpha atom located > 0.8 Å from the C-alpha atom of any residue in the maltogenic alpha-amylase sequence and wherein at least one CGTase residue has a C-alpha atom located <10 Å from a substrate, or is among the three amino acids adjacent to such subsequence in the amino acid sequence,
d) modifying the CGTase sequence wherein the modification comprises substitution or deletion of the selected residue or by insertion of a residue adjacent to the selected residue, and
e) producing the polypeptide having the resulting amino acid sequence.

The invention also provides a variant polypeptide which has an amino acid sequence with at least 70% identity to SEQ ID NO: 6; and has the ability to form linear oligosaccharides as an initial product when acting on starch.

Compared to SEQ ID NO: 6, the variant polypeptide may comprise at least one additional amino acid in a region corresponding to amino acids 194-198 and have a different amino acid or an insertion or deletion at a position corresponding to amino acid 16, 47, 85-95, 117, 139, 145, 146, 152, 153, 168, 169, 174, 184, 191, 260-269, 285, 288, 298, 314, 335, 413, 556, 602 or 677.

Alternatively, compared to SEQ ID NO: 6 the variant polypeptide may comprise at least one additional amino acid in a region corresponding to amino acids 260-269 and have a different amino acid or an insertion or deletion at a position corresponding to amino acid 16, 47, 85-95, 117, 139, 145, 146, 152, 153, 168, 169, 174, 181, 184, 191, 194, 285, 288, 298, 314, 335, 413, 556, 602 or 677.

### BRIEF DESCRIPTION OF DRAWINGS

Fig. 1 shows an alignment of various known CGTase sequences. Details are given below.
Fig. 2 shows the results of a comparison of the 3D structures la47 for a CGTase (SEQ ID NO: 5) and 1qho for the maltogenic alpha-amylase Novamyl (SEQ ID NO: 17). Details are described in Example 1.

### DETAILED DESCRIPTION OF THE INVENTION

### CGTase

The method of the invention uses an amino acid sequence of a CGTase and a three-dimensional model for the CGTase. The CGTase may have a catalytic triad, and the model may include a substrate.

The CGTase may have a three-dimensional structure found under the indicated identifier in the Protein Data Bank (www.rcsb.org): *B. circulans* (1CDG), alkalophilic *Bacillus* (1PAM), *B. stearothermophilus* (1CYG) or *Thermoanaerobacterium thermosulfurigenes* (1CIU, 1A47). 3D structures for other CGTases may be constructed as described in Example 1 of WO 9623874.

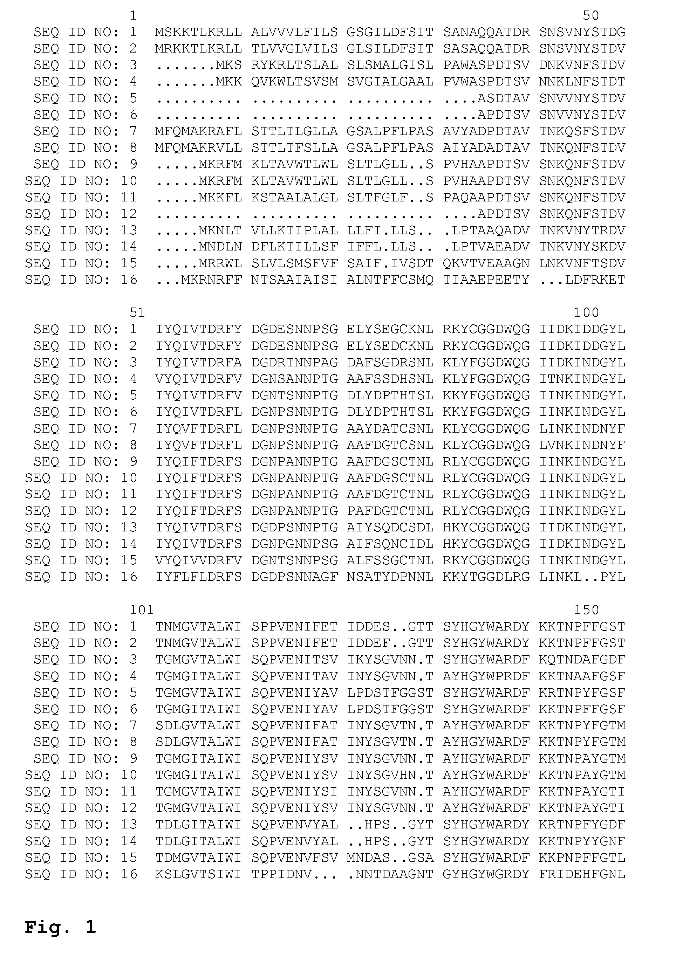

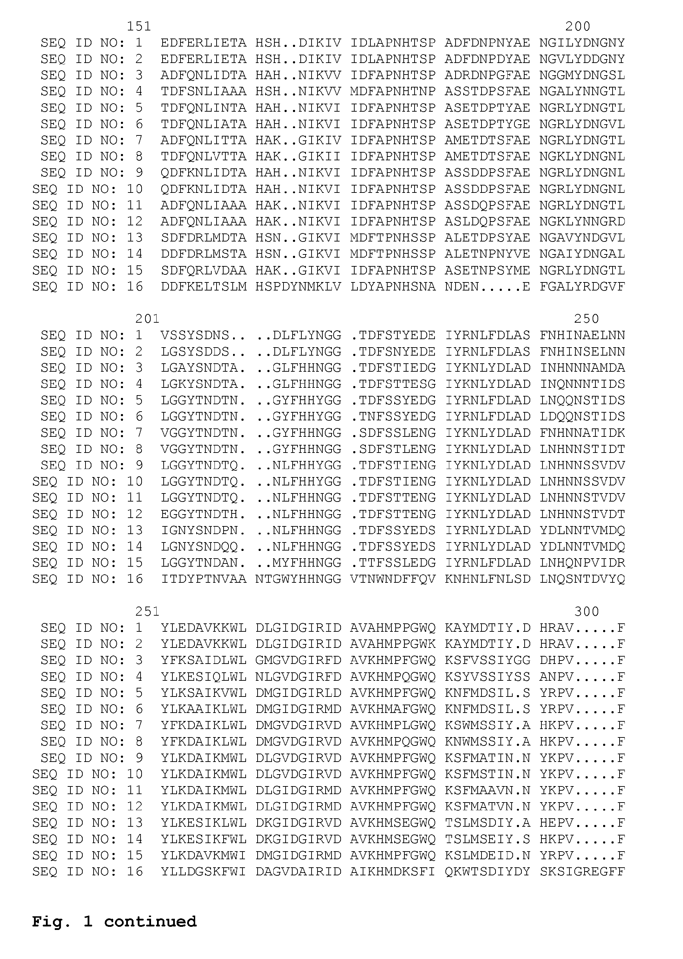

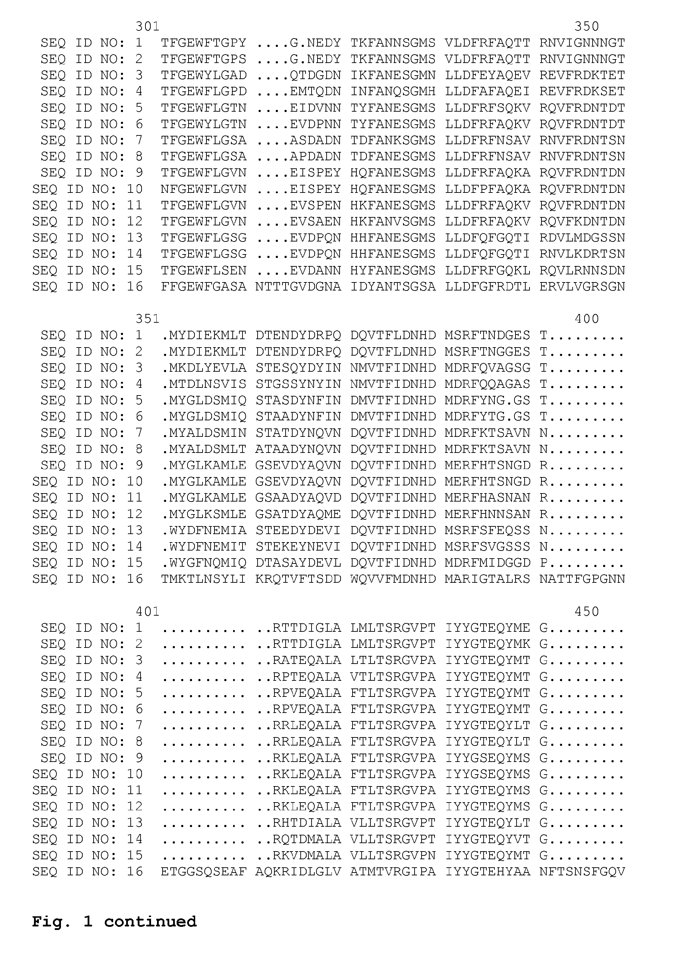

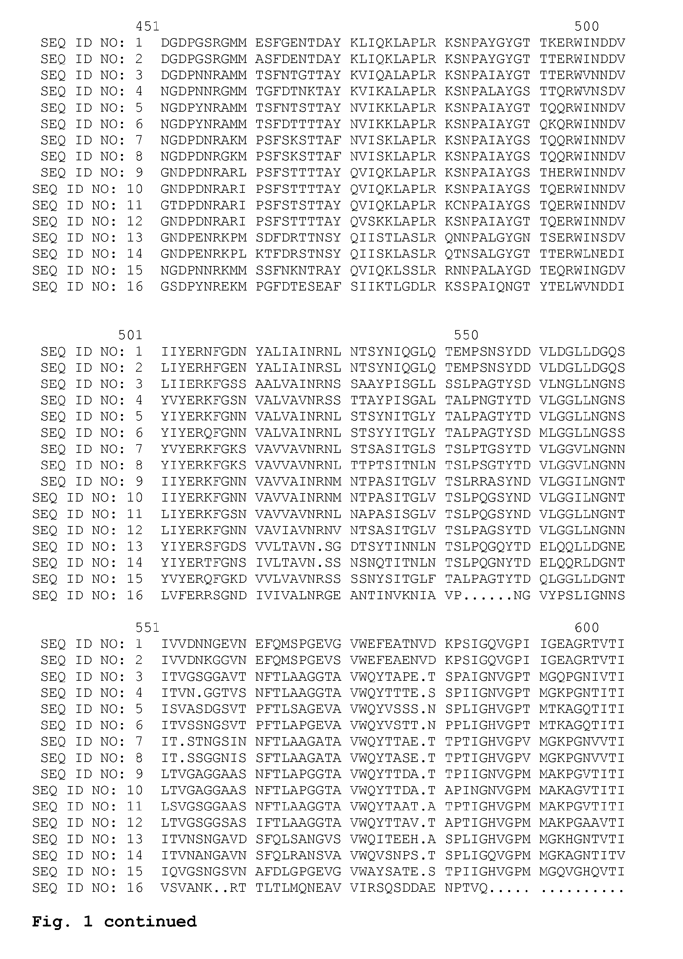

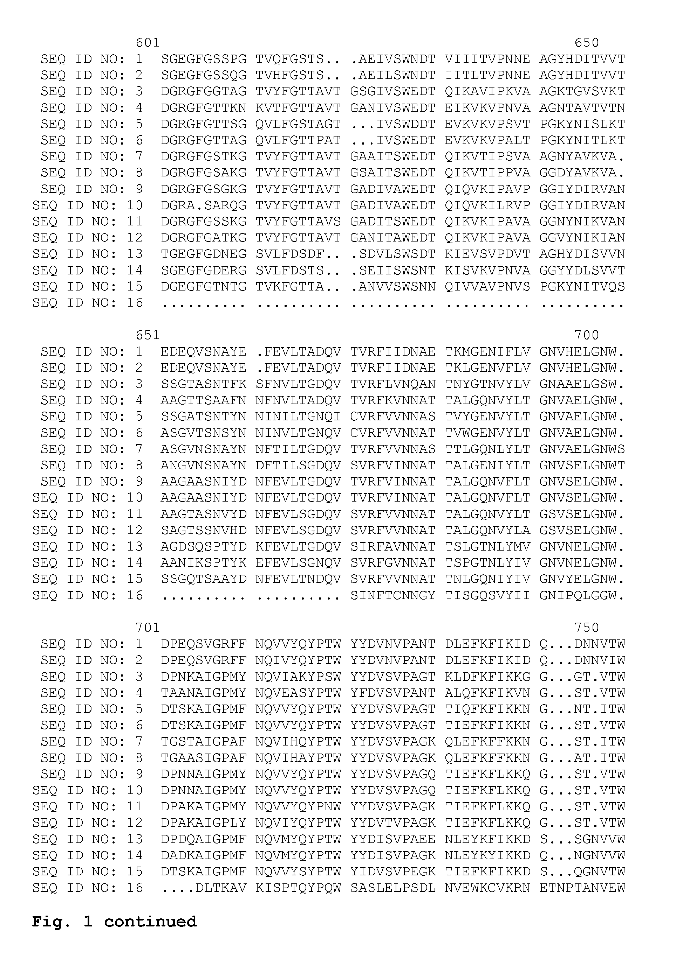

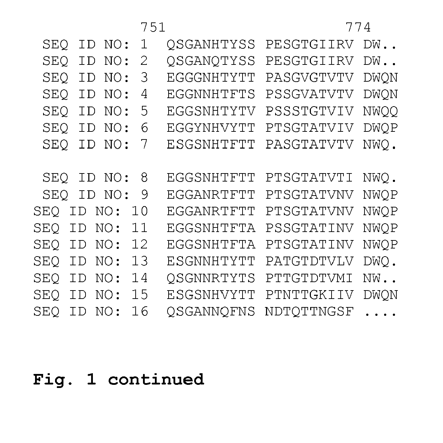

Fig. 1 shows an alignment of the following known CGTase sequences, each identified by accession number in the GeneSeqP database and by source organism. Some sequences include a propeptide, but only the mature peptide is relevant for this invention.
SEQ ID NO: 1. aab71493.gcg *B*. *agaradherens*
SEQ ID NO: 2. aau76326.gcg *Bacillus agaradhaerans*
SEQ ID NO: 3. cdg1_paema.gcg *Paenibacillus macerans (Bacillus macerans).*
SEQ ID NO: 4. cdg2_paema.gcg *Paenibacillus macerans (Bacillus macerans).*
SEQ ID NO: 5. cdgt_thetu.gcg *Thermoanaerobacter thermosulfurogenes (Clostridium thermosulfurogenes)* (SEQ ID NO: 2:)
SEQ ID NO: 6. aaw06772.gcg *Thermoanaerobacter thermosulphurigenes* sp. ATCC 53627 (SEQ ID NO: 3)
SEQ ID NO: 7. cdgt_bacci.gcg *Bacillus circulans*
SEQ ID NO: 8. cdgt_bacli.gcg *Bacillus sp.* (strain 38-2)
SEQ ID NO: 9. cdgt_bacs0.gcg *Bacillus sp.* (strain 1011)
SEQ ID NO: 10. cdgt_bacs3.gcg *Bacillus sp.* (strain 38-2)
SEQ ID NO: 11 cdgu_bacci.gcg *Bacillus circulans*
SEQ ID NO: 12. cdgt_bacsp.gcg *Bacillus sp.* (strain 17-1, WO 2003068976) (SEQ ID NO: 4)
SEQ ID NO: 13. cdgt_bacoh.gcg *Bacillus ohbensis*
SEQ ID NO: 14. cdgt_bacs2.gcg *Bacillus sp.* (strain 1-1)
SEQ ID NO: 15. cdgt_bacst.gcg *Bacillus stearothermophilus*
SEQ ID NO: 16. cdgt_klepn.gcg *Klebsiella pneumoniae*

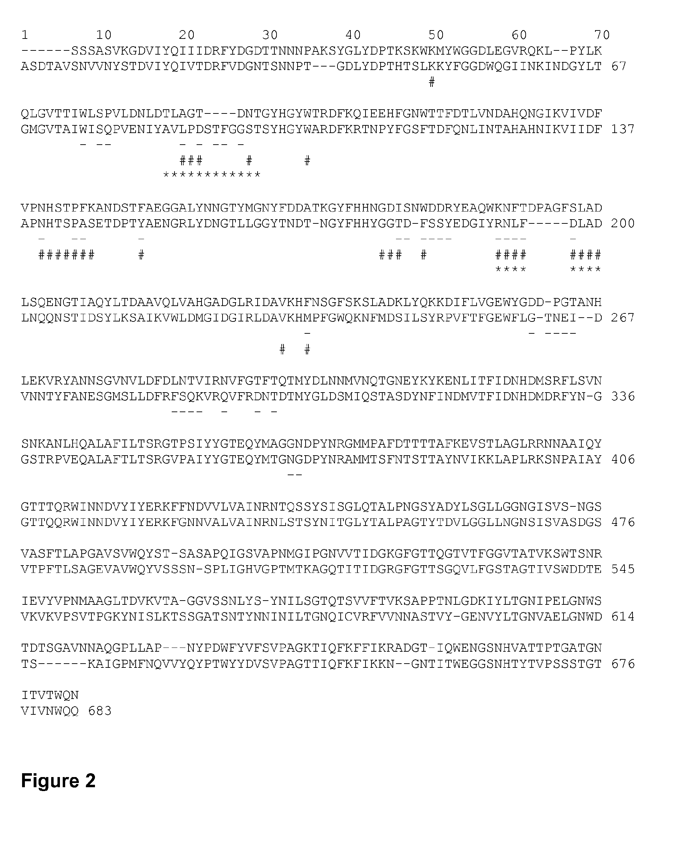

To develop variants of a CGTase without a known 3D structure, the sequence may be aligned with a CGTase having a known 3D structure. An alignment for a number of CGTase sequences is shown in Fig. 2. Other sequences may be aligned by conventional methods, e.g. by use the software GAP from UWGCG Version 8.

### Maltogenic alpha-amylase

The method also uses an amino acid sequence of a maltogenic alpha-amylase (EC 3.2.1.133) and a three-dimensional model of the maltogenic alpha-amylase. The maltogenic alpha-amylase may have a catalytic triad, and the model may include a substrate. The maltogenic alpha-amylase may have the amino acid sequence shown in SEQ ID NO: 17 (in the following referred to as Novamyl). A 3D model for Novamyl with a substrate is described in US 6162628 and is found in the Protein Data Bank with the identifier 1QHO. Alternatively, the maltogenic alpha-amylase may be a Novamyl variant described in US 6162628. A 3D structure of such a variant may be developed from the Novamyl structure by known methods, e.g. as described in T.L. Blundell et al., Nature, vol. 326, p. 347 ff (26 March 1987); J. Greer, Proteins: Structure, Function and Genetics, 7:317-334 (1990); or Example 1 of WO 9623874.

### Superimposition of 3D models

The two 3D models may be superimposed by aligning the amino acid residues of each catalytic triad. This may be done by methods known in the art based on the deviations of heavy atoms in the two triads, e.g. by minimizing the sum of squares of deviations. Alternatively, the superimposition may be done so as to keep deviations between corresponding atoms below 0.8 Å, e.g. below 0.6 Å, below 0.4 Å" below 0.3 Å or below 0.2 Å.

Alternatively, the superimposition may be based on the deviations of all corresponding pairs of amino acid residues as shown in the alignment in Figs. 4-5 of WO 9943793 and bringing the sum of square of all deviations to a minimum.

### Selection of amino acid sequences

In the superimposed 3D models, amino acid residues in the CGTase sequence are selected if they meet at least one of three conditions:
■ The CGTase residue has a C-alpha atom located > 0.8 Å from the C-alpha atom of any amino acid residue in the maltogenic alpha-amylase, and it is located < 10 Å from an atom of a substrate.
■ The CGTase residue has a C-alpha atom located < 6 Å from a heavy atom (i.e., an atom other than H) of an amino acid residue of the maltogenic alpha-amylase corresponding to residue 190-194 of SEQ ID NO: 17.
■ The CGTase residue is in a subsequence (a "loop") of the CGTase or in the "pre-fix" or "post-fix" of the loop. The CGTase loop is a subsequence wherein each residue has a C-alpha atom located > 0.8 Å from the C-alpha atom of any residue in the maltogenic alpha-amylase sequence, and at least one CGTase residue of the loop has a C-alpha atom located <10 Å from a substrate. The pre-fix and post-fix are defined as three amino acid residues in the sequence before and after the loop.

The selected CGTase residue may correspond to residue 47, 75, 77, 78, 85, 86, 87, 88, 89, 90, 91, 92, 93, 94, 95, 96, 102, 139, 140, 141, 142, 143, 144, 145, 146, 152, 153, 168, 169, 180, 181, 182, 183, 184, 185, 186, 187, 191, 193, 194, 195, 196, 197, 198, 199, 200, 231, 234, 235, 262, 263, 264, 265, 266, 286, 287, 288, 289, 292, 296, 298, 335, 353, 369, 370, 413, or 556 of SEQ ID NO: 5.

### Modifications of CGTase amino acid sequence

A selected CGTase residue may be deleted or may be substituted with a different residue. The substitution may be made with the same amino acid residue as found at a corresponding position in an alignment with the maltogenic alpha-amylase sequence or with a residue of the same type. The type indicates a positively charged, negatively charged, hydrophilic or hydrophobic residue, understood as follows (Tyr may be hydrophilic or hydrophobic):
Hydrophobic amino acids: Ala, Val, Leu, Ile, Pro, Phe, Trp, Gly, Met, Tyr
Hydrophilic amino acids: Thr, Ser, Gln, Asn, Tyr, Cys
Positively charged amino acids: Lys, Arg, His
Negatively charged amino acids: Glu, Asp

The substitution of the CGTase residue may be with a larger or smaller residue depending on whether a larger or smaller residue is found at a corresponding position in the maltogenic alpha-amylase sequence. In this connection, the residues are ranked as follows from smallest to largest: (an equal sign indicates residues with sizes that are practically indistinguishable):
G < A=S=C < V=T < P < L=I=N=D=M < E=Q < K < H < R < F < Y < W

One or more amino acid residues may be inserted at a position adjacent to the selected CGTase residue on the amino or carboxyl side. The insertion may be made at a position in the CGTase sequence where the maltogenic amylase contains additional residues, and the insertion may consist of an equal number of residues, or the insertion may have one or two fewer or more residues. Each inserted residue may be the same as the corresponding maltogenic amylase residue or of the same type.

The insertion may particularly be made at a position corresponding to residues in the regions 85-96, 193-200 or 260-269 of SEQ ID NO: 5. The insertion at residues 193-200 may particularly consist of 1-7 residues, e.g. 1, 2, 3, 4, 5, 6 or 7 residues, and may particularly consist of DPAGF, e.g. between residues 196 and 197 of SEQ ID NO: 5, and it may be combined with a substitution corresponding to L195F, F196T and D197S in SEQ ID NO: 5.

More particularly, the modification may comprise substitution of amino acids corresponding to amino acids 85-95, 260-268 or 260-269 of SEQ ID NO: 5 or 6 with TLAGTDN, YGDDPGTANHL or YGDDPGTANHLE, respectively.

The substitution may correspond to V16A, K47K, T117R, P139L, A145F, F146K, Y152F, G153V/G, Y168F, T169I, G174S, G181D, F184W, I191T, N194S, R285D, Q288T, T2981, D314E, T335A, R353H, W413R, G556S, Y602L, or V677K of SEQ ID NO: 5 or 6.

### Optional further modifications of the CGTase sequence

Optionally, the CGTase sequence may be further modified by substituting one or more residues which is not selected. The substitution may be made with an amino acid residue of the same type (in particular with the same residue) as the corresponding residue in an alignment with the maltogenic alpha-amylase sequence.

Depending on whether the matching residue in the maltogenic alpha-amylase sequence is smaller or larger than the residue in the CGTase sequence, the substitution may be made with a smaller or larger residue (using the ranking shown above).

### Production of CGTase variants

A polypeptide having the resulting amino acid sequence may be produced by conventional methods, generally involving producing DNA with a sequence encoding the polypeptide together with control sequences, transforming a suitable host organism with the DNA , cultivating the transformed organism at suitable conditions for expressing and optionally secreting the polypeptide, and optionally recovering the expressed polypeptide, e.g. as described in WO 9943793.

DNA encoding any of the above CGTase variants may be prepared, e.g. by point-specific mutation of DNA encoding the parent CGTase. This may be followed by transformation of a suitable host organism with the DNA, and cultivation of the transformed host organism under suitable conditions to express the encoded polypeptide (CGTase variant). This may be done by known methods.

### Properties of CGTase variants

The CGTase variants of the invention may form linear oligosaccharides as an initial product by starch hydrolysis and a reduced amount of cyclodextrin and may be useful for anti-staling in baked products. The modification of the amino acid sequence according to the invention may result in reduced cyclization and disproportionation activities and an increased ratio of hydrolysis/cyclization activities, measured, e.g., as described by H. Leemhuis, Journal of Biotechnology, 103 (2003), 203-212.

Optionally, one or more expressed polypeptides may be tested for one or more useful enzymatic activities, and a variant may be selected accordingly. Thus, the ability to hydrolyze starch or a starch derivative may be tested by a conventional method, e.g. a plate assay, use of Phadebas tablets or DSC on amylopectin. Further, the initial product from starch hydrolysis may be analyzed and a polypeptide producing an increased ratio of linear oligosaccharides to cyclodextrins may be selected. The initial product may have a high ratio of maltose or maltose + glucose (G2 or G1+G2) compared to total dextrins (maltooligosaccharides G1-G7 or G1-G7 + cyclodextrins). This may be measured as described in an example.

Also, the polypeptide may be tested by adding it to a dough, baking it and testing the firmness of the baked product during storage; a polypeptide with anti-staling effect may be selected as described in WO 9104669 or US 6162628.

The substitutions according to the invention may improve the thermostability of the CGTase variants. Variants may be screened for their thermostability, e.g. by DSC (differential scanning calorimetry) at pH 5.5 in 0.1 M Na acetate, scan rate 90 K/h, and a variant with an improved thermostability may be selected. The substitutions may also increase the yield when expressed in a suitable transformed host organism; this may be edxplained by an improved stability.

Optionally, the amino acid sequence may be further modified to improve the properties of the variant, particularly to improve its thermostability. Such modification may include amino acid substitutions similar to those described in US 6162628 or in H. Leemhuis et al., Proteins: Structure, Function and Bioinformatics, 54:128-134 (2004).

### Optional gene recombination

Optionally, DNA encoding a plurality of the above CGTase variants may be prepared and recombined, followed by transformation of a suitable host organism with the recombined DNA, and cultivation of the transformed host organism under suitable conditions to express the encoded polypeptides (CGTase variants). The gene recombination may be done by known methods.

### CGTase variants

Particularly, the CGTase may be modified by substitution, insertion or deletion of an amino acid at a position corresponding to amino acid 85-95, 152, 184, 260-269, 285, 288, 314 of the amino acid sequence shown in SEQ ID NO: 5 or 6. The modification may comprise substitution or insertion of an amino acid residue with an amino acid residue of a corresponding position in the amino acid sequence of Novamyl (SEQ ID NO: 17) or a deletion of an amino acid residue in the region which is not present at the corresponding position in the Novamyl sequence.

More particularly, the modification may comprise substitution of amino acids corresponding to amino acids 85-95, 260-268 or 260-269 of SEQ ID NO: 5 or 6 with TLAGTDN, YGDDPGTANHL or YGDDPGTANHLE, respectively.

Some particular examples with the *Thermoanaerobacter* CGTase (SEQ ID NO: 6) as an example are Y152F, F184W, R285D, Q288T, D314E. Corresponding substitutions may be made in other CGTases.

Also, one or more additional modifications may be made, each being an amino acid substitution, insertion or deletion. In particular, such modification may be made in the regions corresponding to amino acids 40-43, 78-85, 136-139, 173-180, 189-195 or 258-268 of SEQ ID NO: 17. In particular, the modification may be an insertion of or a substitution with an amino acid present at the corresponding position of Novamyl, or a deletion of an amino acid not present at the corresponding position of Novamyl. Thus, taking the *Thermoanaerobacter* CGTase (SEQ ID NO: 6) as an example, one or more of the following changes may be made to introduce a loop modeled on Novamyl:
- A85-S95 of SEQ ID NO: 6 is replaced by T80-N86 of SEQ ID NO: 17,
- N194-L198 of SEQ ID NO: 6 is replaced by N187-L196 of SEQ ID NO: 17,
- Y260-P268 of SEQ ID NO: 6 is replaced by Y258-L268 of SEQ ID NO: 17, or
- Y260-N269 of SEQ ID NO: 6 is replaced by Y258-E269 of SEQ ID NO: 17.

### EXAMPLES

### Example 1: Construction of CGTase residues based on 3D structures

Two 3D structures with substrates were used: 1A47 for a CGTase (SEQ ID NO: 5) and 1 QHO for a maltogenic alpha-amylase (Novamyl, SEQ ID NO: 17), wherein the substrates are indicated as GTE, GLC, CYL and GLD for 1 a47 and as ABD for 1 qho. The two structures were superimposed by minimizing the sum of squares for deviations at the three C-alpha atoms at the catalytic triad: D230, E258 and D329 for 1A47, and D228, E256 and D329 for Novamyl. The superimposed structures were analyzed, and the result is shown in Fig. 2 with the Novamyl sequence at the top and the CGTase sequence below.

The following CGTase residues were found to have a C-alpha atom < 10 Å from an atom of either substrate: 19, 21, 24, 46-47, 75, 77-78, 82-83, 85-103, 106, 136-145, 152-153, 182-187, 190-191, 193-200, 228-235, 257-267, 270, 282-289, 291-292, 296, 298, 324, 327-331, 359, 369-375. Out of these, the following were found to have a C-alpha atom > 0.8 Å from the C-alpha atom of any Novamyl residue: 75, 77-78, 87, 89, 91-92, 94, 140, 144-145, 152, 182-187, 193-197, 235, 262-266, 286-289, 292, 296, 298, 369-370. They are indicated by underlining in Fig. 2.

The following CGTase residues were found to have a C-alpha atom < 6 Å from an atom other than hydrogen (a "heavy" atom) of one of the Novamyl residues 190-194: 47, 87-89, 95, 102, 140-146, 152, 180-182, 184, 193-200, 231, 234. They are marked by # in Fig. 2.

Two subsequences ("loops") of consecutive CGTase residues were identified where some residues have the C-alpha atom < 10 Å from an atom of either substrate and > 0.8 Å from the C-alpha atom of any Novamyl residue. Including prefix and postfix (3 residues each), the two subsequences are at residues 85-96 and 193-200 of the CGTase. They are indicated by asterisks in Fig. 2.

To construct variants of the CGTase of SEQ ID NO: 6, the corresponding residues were identified in the alignment in Fig. 1. As a result of the high degree of identity, the residues have the same numbers in the two sequences. Variants were constructed, each having one or more loops modeled on Novamyl together with one or more substitutions, as follows:
Novamyl T80-N86: 85A*, 86V*, 87L*, 88P*, D89T, S90L, T91A, F92G, G93T, G94D
Novamyl G259-L268: *260aG, *260bD, L261D, G262P, T263G, N264T, E265A, V266N, D267H, P268L
Novamyl F188-S195: *194aF, *194bT, *194cD, *194dP, *194eA, L195G, D197S

| Novamyl loops | Additional substitutions |
|---|---|
| T80-N86, F188-S195 | Y152F |
| T80-N86, F188-S195, G259-L268 | Y152F , D314E |
| T80-N86, F188-S195, G259-L268 | Y152F, F184W, R285D, Q288T, D314E |
| T80-N86, G259-L268 | Y152F, G257D, R285D, Q288T, D314E |
| T80-N86, G259-L268 | Y152F, R285D, Q288T, D314E |
| T80-N86, G259-L268 | A145F, Y152F, R285D, Q288T, D314E |
| T80-N86, G259-L268 | S146K, Y152F, R285D, Q288T, D314E |
| T80-N86, G259-L268 | A145F, S146K, Y152F, G257D, R285D, Q288T, D314E |
| T80-N86, F188-S195, G259-L268 | A145F, Y152F, F184W, R285D, Q288T, D314E |
| T80-N86, F188-S195, G259-L268 | S146K, Y152F, F184W, R285D, Q288T, D314E |
| T80-N86, F188-S195, G259-L268 | A145F, S146K, Y152F, F184W, R285D, Q288T, D314E |
| T80-N86 | Y152F, T207N |
| T80-N86, G259-L268 | A145F, Y152F, R285D, Q288T, D314E |
| T80-N86, G259-L268 | S146K, Y152F, R285D, Q288T, D314E |
| T80-N86, G259-L268 | A145F, S146K, Y152F, R285D, Q288T, D314E |
| T80-N86, G259-L268 | Y152F, F196G, G257D, R285D, Q288T, D314E |
| T80-N86, G259-L268 | Y152F, F196G, R285D, Q288T, D314E |
| T80-N86, G259-L268 | Y152F, F184N, F196G, G257D, R285D, Q288T, D314E |
| T80-N86, G259-L268 | Y152F, F184N, F196G, R285D, Q288T, D314E |
| T80-N86, F188-S195, G259-L268 | Y152F, R285D, Q288T, D314E |
| T80-N86, F188-S195, G259-L268 | A145F, Y152F, R285D, Q288T, D314E |
| T80-N86, F188-S195, G259-L268 | S146K, Y152F, R285D, Q288T, D314E |
| T80-N86, F188-S195, G259-L268 | A145F, S146K, Y152F, R285D, Q288T, D314E |
| T80-N86, F188-S195, G259-L268 | Y152F, G181D, F184W, R285D, Q288T, D314E |
| T80-N86, G259-L268 | Y152F, G181D, F184W, G257D, R285D, Q288T, D314E |
| T80-N86, G259-L268 | A145F, Y152F, G181D, F184W, R285D, Q288T, D314E |
| T80-N86, G259-L268 | S146K, Y152F, G181D, F184W, R285D, Q288T, D314E |
| T80-N86, G259-L268 T80-N86, G259-L268 | A145F, S146K, Y152F, G181D, F184W, G257D, R285D, Q288T, D314E |
| T80-N86, F188-S195, G259-L268 | A145F, Y152F, G181D, F184W, R285D, Q288T, D314E |
| T80-N86, F188-S195, G259-L268 | S146K, Y152F, G181D, F184W, R285D, Q288T, D314E |
| T80-N86, F188-S195, G259-L268 | A145F, S146K, Y152F, G181D, F184W, R285D, Q288T, D314E |
| T80-N86, F188-S195, G259-L268 | Y152F, G181D, R285D, Q288T, D314E |
| T80-N86, F188-S195, G259-L268 | A145F, Y152F, G181D, R285D, Q288T, D314E |
| T80-N86, F188-S195, G259-L268 | S146K, Y152F, G181D, R285D, Q288T, D314E |
| T80-N86, F188-S195, G259-L268 | A145F, S146K, Y152F, G181D, R285D, Q288T, D314E |
| T80-N86, G259-L268 | Y152F, G181D G257D, R285D, Q288T, D314E |
| T80-N86, G259-L268 | A145F, Y152F, G181D, R285D, Q288T, D314E |
| T80-N86, G259-L268 | S146K, Y152F, G181D, R285D, Q288T, D314E |
| T80-N86, G259-L268 | A145F, S146K, Y152F, G181D, G257D, R285D, Q288T, D314E |
| T80-N86, F188-S195, G259-L268 | A145F, S146K, Y152F, G181D, F184W, R285D, Q288T, D314E, F384S |

Similarly, variants of the CGTase of SEQ ID NO: 12 were constructed, each having modifications to emulate the following three Novamyl loops:
T80-D85: 85S*, 86V*, 871*, N88T, Y89L, S90A, V92T, N93D
F188-S195: L194F, Y195T, *196aP, *196bA, *196cG, *196dF, *196eS
Y258-L268: "258aY, *258bG, F259D, L260D, G261P, V262G, N263T, E264A, 1265N, S266H, P267L"

| Novamyl loops | Additional substitutions |
|---|---|
| T80-D85, F188-S195, Y258-L268 | N173S |
| T80-D85, F188-S195, Y258-L268 | R284D, Q287T, D313E, F605L |
| T80-D85, F188-S195, Y258-L268 | Q116R, D639G |
| T80-D85, F188-S195, Y258-L268 | V16A, Q116R, A144F, S145K, R284D, Q287T, M680K |
| T80-D85, F188-S195, Y258-L268 | A144F, S145K, R284D, Q287T, D313E |
| T80-D85, F188-S195, Y258-L268 | A144F, S145K, G180D, R284D, Q287T, D313E |
| T80-D85, F188-S195, Y258-L268 | A144F, S145K, G180D, F183W, R284D, Q287T, D313E |
| T80-D85, F188-S195, Y258-L268 | A144F, S145K, F183W, R284D, Q287T, D313E |
| T80-D85, F188-S195, Y258-L268 | R47K, A144F, S145K, R284D, Q287T, D313E |
| T80-D85, F188-S195, Y258-L268 | R47K, A144F, S145K, G180D, R284D, Q287T, D313E |
| T80-D85, F188-S195, Y258-L268 | R47K, A144F, S145K, G180D, F183W, R284D, Q287T, D313E |
| T80-D85, F188-S195, Y258-L268 | R47K, A144F, S145K, F183W, R284D, Q287T, D313E |
| T80-D85, F188-S195, Y258-L268 | Q116R, P138L, A144F, S145K, A152V, 1190T, T334A, R353H |
| T80-D85, F188-S195, Y258-L268 | A144F, S145K, Y167F, T1681, N173S, N193S, T2971, G559S |
| T80-D85, F188-S195, Y258-L268 | A144F, S145K, A152G, W413R, F605L |

### Example 2: Starch hydrolysis with CGTase variants

Nine variants prepared in Example 1 were tested to determine the initial product profile in starch hydrolysis. The variants including 7 variants of SEQ ID NO: 6 and 2 variants of SEQ ID NO: 12. The two parent CGTases were tested for comparison.

Incubations were carried out using 2% amylopectin (potato starch) in 50 mM NaOAc, pH 5.7, 5 mM CaCl2. Crude culture broth (20-100 micro-L) was added to the substrate solution (900-980 micro-L), and the mixture incubated at 40°C or 60°C and the conversion was followed by TLC (TLC eluent: acetonitrile/EtOAc, n-propanol/water 85:20:50:50, visualization: 1M H₂SO₄ followed by heating). At a detectable conversion (4-18 h), a sample (100 micro-L) was taken out and inactivated with 1 M NaOH (10 micro-L). The sample was diluted (30 micro-L to 1000 micro-L MilliQ water) and filtered through 0.45 µm Millex®-HV filter before analysis by HPAEC /high-performance anion exchange chromatography).

The samples were analyzed on a Dionex DX-500 HPAEC-PAD system (CarboPac PA-100 column; A buffer: 150 mM NaOH; B buffer: 150 mM NaOH + 0.6 M sodium acetate; Flow rate: 1 ml/min. Elution conditions: 0-3 min: 95% A + 5% B; 3-19 min: linear gradient: 95% A+ 5% B to 50% A and 50% B; 19-21 min: linear gradient: 50% A + 50% B to 100% B; 21-23 min: 100% B). As reference on the Dionex system a mixture of maltooligosaccharides was used (DP2 to DP7, 100 micro-M of each) and α-, β- and γ-CD (100 micro-M of each). These were used to quantify the amounts of each oligosaccharide formed.

The results were expressed as G2/sum, (G1+G2)/sum and CD/sum where G1 is the peak area for glucose, G2 is the peak area for maltose, CD is the total of peak areas for alpha-, beta- and gamma-cyclodextrin, and sum is the total of peak areas for G1-G7 maltodextrins and mcyclodextrins. G2/sum was 0.12-0.68 for the variants compared to 0 or 0.03 for the parent CGTases. (G1+G2)/sum was 0.48-0.79 for the variants compared to 0 and 0.06 for the parent CGTases. CD/sum was 0.01-0.18 for the variants compared to 0.87 and 0.94 for the parent CGTases.

### Example 3: Baking tests with CGTase variants

Ten variants prepared in Example 1 were purified and tested in baking, including 7 variants of SEQ ID NO: 6 and 3 variants of SEQ ID NO: 12. Doughs were made according to the straight-dough method with addition of the CGTase variant at a dosage in the range of 1-20 mg/kg. Controls were made without enzyme addition or with addition of one of the two parent CGTases.

The doughs were baked to make panned bread, and the bread was stored for a week. Firmness, elasticity and mobility of free water were measured for the bread loaves after 1, 4 and 7 days storage. A sensory ranking of moistness was made by a trained test panel for bread after 7 days.

Each of the variants was ranked better than a control without enzyme. The CGTases had a detrimental effect on elasticity, whereas the variants did not effect the elasticity negatively. The bread made with CGTase was gummy and unacceptable.

## Claims

1. A polypeptide which:
a) has an amino acid sequence having at least 70% identity to SEQ ID NO: 6;
b) compared to SEQ ID NO: 6 comprises at least one additional amino acid in a region corresponding to amino acids 194-198,
c) compared to SEQ ID NO: 6 has a different amino acid or an insertion or deletion at a position corresponding to amino acid 16, 117, 139, 168, 169, 174, 285, 288, 298, 314, 335, 413, 556, 602 or 677, and
d) has the ability to form linear oligosaccharides as an initial product when acting on starch.

2. A polypeptide which:
a) has an amino acid sequence having at least 70% identity to SEQ ID NO: 6;
b) compared to SEQ ID NO: 6 comprises at least one additional amino acid in a region corresponding to amino acids 260-269,
c) compared to SEQ ID NO: 6 has a different amino acid or an insertion or deletion at a position corresponding to amino acid 16, 85-95, 117, 139, 168, 169, 174, 181, 285, 288, 298, 314, 335, 413, 556, 602 or 677, and
d) has the ability to form linear oligosaccharides as an initial product when acting on starch.

3. The polypeptide of claim 1 or 2 which compared to SEQ ID NO: 6 comprises 1-7 additional amino acids in a region corresponding to amino acids 194-198, particularly 5 amino acids, more particularly insertion of DPAGF, most particularly between amino acids corresponding to 196 and 197 of SEQ ID NO: 6.

4. The polypeptide of any of claims 1-3, which has a different amino acid from SEQ ID NO: 6 at a position corresponding to 194-198, particularly F at a position corresponding to L195 of SEQ ID NO: 6, T at F196 or S at D197.

5. The polypeptide of any of claims 1-3, which comprises an amino acid residue which is present at the corresponding position of SEQ ID NO: 17 or deletion of an amino acid residue in SEQ ID NO: 6 which is not present at the corresponding position in the amino acid sequence shown in SEQ ID NO: 17.

6. The polypeptide of any of claims 1-3, which has TLAGTDN at positions corresponding to 85-95 of SEQ ID NO: 6, YGDDPGTANHL at 260-268 or YGDDPGTANHLE at 260-269.

7. The polypeptide of any of claims 1-5 which compared to SEQ ID NO: 6 has a substitution corresponding to V16A, T117R, P139L, Y168F, T1691, G174S, G181D, N194S, R285D, Q288T, T2981, D314E, T335A, R353H, W413R, G556S, Y602L, V677K.

8. A polynucleotide encoding the polypeptide of any of claims 1-7.

9. A process for preparing a baked product which comprises adding the polypeptide of any of claims 1-7 to a dough and baking the dough to prepare the baked product.


SEQUENCE LISTING

<110>  Novozymes A/S

<120>  CGTASE VARIANTS

<130>  10340-WO

<160>  17

<170>  PatentIn version 3.2

<210>  1
<211>  713
<212>  PRT
<213>  Bacillus agaradherens

<400>  1

Met Ser Lys Lys Thr Leu Lys Arg Leu Leu Ala Leu Val Val Val Leu
1               5                   10                  15

Phe Ile Leu Ser Gly Ser Gly Ile Leu Asp Phe Ser Ile Thr Ser Ala
            20                  25                  30

Asn Ala Gln Gln Ala Thr Asp Arg Ser Asn Ser Val Asn Tyr Ser Thr
        35                  40                  45

Asp Gly Ile Tyr Gln Ile Val Thr Asp Arg Phe Tyr Asp Gly Asp Glu
    50                  55                  60

Ser Asn Asn Pro Ser Gly Glu Leu Tyr Ser Glu Gly Cys Lys Asn Leu
65                  70                  75                  80

Arg Lys Tyr Cys Gly Gly Asp Trp Gln Gly Ile Ile Asp Lys Ile Asp
                85                  90                  95

Asp Gly Tyr Leu Thr Asn Met Gly Val Thr Ala Leu Trp Ile Ser Pro
            100                 105                 110

Pro Val Glu Asn Ile Phe Glu Thr Ile Asp Asp Glu Ser Gly Thr Thr
        115                 120                 125

Ser Tyr His Gly Tyr Trp Ala Arg Asp Tyr Lys Lys Thr Asn Pro Phe
    130                 135                 140

Phe Gly Ser Thr Glu Asp Phe Glu Arg Leu Ile Glu Thr Ala His Ser
145                 150                 155                 160

His Asp Ile Lys Ile Val Ile Asp Leu Ala Pro Asn His Thr Ser Pro
                165                 170                 175

Ala Asp Phe Asp Asn Pro Asn Tyr Ala Glu Asn Gly Ile Leu Tyr Asp
            180                 185                 190

Asn Gly Asn Tyr Val Ser Ser Tyr Ser Asp Asn Ser Asp Leu Phe Leu
        195                 200                 205

Tyr Asn Gly Gly Thr Asp Phe Ser Thr Tyr Glu Asp Glu Ile Tyr Arg
    210                 215                 220

Asn Leu Phe Asp Leu Ala Ser Phe Asn His Ile Asn Ala Glu Leu Asn
225                 230                 235                 240

Asn Tyr Leu Glu Asp Ala Val Lys Lys Trp Leu Asp Leu Gly Ile Asp
                245                 250                 255

Gly Ile Arg Ile Asp Ala Val Ala His Met Pro Pro Gly Trp Gln Lys
            260                 265                 270

Ala Tyr Met Asp Thr Ile Tyr Asp His Arg Ala Val Phe Thr Phe Gly
        275                 280                 285

Glu Trp Phe Thr Gly Pro Tyr Gly Asn Glu Asp Tyr Thr Lys Phe Ala
    290                 295                 300

Asn Asn Ser Gly Met Ser Val Leu Asp Phe Arg Phe Ala Gln Thr Thr
305                 310                 315                 320

Arg Asn Val Ile Gly Asn Asn Asn Gly Thr Met Tyr Asp Ile Glu Lys
                325                 330                 335

Met Leu Thr Asp Thr Glu Asn Asp Tyr Asp Arg Pro Gln Asp Gln Val
            340                 345                 350

Thr Phe Leu Asp Asn His Asp Met Ser Arg Phe Thr Asn Asp Gly Glu
        355                 360                 365

Ser Thr Arg Thr Thr Asp Ile Gly Leu Ala Leu Met Leu Thr Ser Arg
    370                 375                 380

Gly Val Pro Thr Ile Tyr Tyr Gly Thr Glu Gln Tyr Met Glu Gly Asp
385                 390                 395                 400

Gly Asp Pro Gly Ser Arg Gly Met Met Glu Ser Phe Gly Glu Asn Thr
                405                 410                 415

Asp Ala Tyr Lys Leu Ile Gln Lys Leu Ala Pro Leu Arg Lys Ser Asn
            420                 425                 430

Pro Ala Tyr Gly Tyr Gly Thr Thr Lys Glu Arg Trp Ile Asn Asp Asp
        435                 440                 445

Val Ile Ile Tyr Glu Arg Asn Phe Gly Asp Asn Tyr Ala Leu Ile Ala
    450                 455                 460

Ile Asn Arg Asn Leu Asn Thr Ser Tyr Asn Ile Gln Gly Leu Gln Thr
465                 470                 475                 480

Glu Met Pro Ser Asn Ser Tyr Asp Asp Val Leu Asp Gly Leu Leu Asp
                485                 490                 495

Gly Gln Ser Ile Val Val Asp Asn Asn Gly Glu Val Asn Glu Phe Gln
            500                 505                 510

Met Ser Pro Gly Glu Val Gly Val Trp Glu Phe Glu Ala Thr Asn Val
        515                 520                 525

Asp Lys Pro Ser Ile Gly Gln Val Gly Pro Ile Ile Gly Glu Ala Gly
    530                 535                 540

Arg Thr Val Thr Ile Ser Gly Glu Gly Phe Gly Ser Ser Pro Gly Thr
545                 550                 555                 560

Val Gln Phe Gly Ser Thr Ser Ala Glu Ile Val Ser Trp Asn Asp Thr
                565                 570                 575

Val Ile Ile Ile Thr Val Pro Asn Asn Glu Ala Gly Tyr His Asp Ile
            580                 585                 590

Thr Val Val Thr Glu Asp Glu Gln Val Ser Asn Ala Tyr Glu Phe Glu
        595                 600                 605

Val Leu Thr Ala Asp Gln Val Thr Val Arg Phe Ile Ile Asp Asn Ala
    610                 615                 620

Glu Thr Lys Met Gly Glu Asn Ile Phe Leu Val Gly Asn Val His Glu
625                 630                 635                 640

Leu Gly Asn Trp Asp Pro Glu Gln Ser Val Gly Arg Phe Phe Asn Gln
                645                 650                 655

Val Val Tyr Gln Tyr Pro Thr Trp Tyr Tyr Asp Val Asn Val Pro Ala
            660                 665                 670

Asn Thr Asp Leu Glu Phe Lys Phe Ile Lys Ile Asp Gln Asp Asn Asn
        675                 680                 685

Val Thr Trp Gln Ser Gly Ala Asn His Thr Tyr Ser Ser Pro Glu Ser
    690                 695                 700

Gly Thr Gly Ile Ile Arg Val Asp Trp
705                 710

<210>  2
<211>  713
<212>  PRT
<213>  Bacillus agaradherens

<400>  2

Met Arg Lys Lys Thr Leu Lys Arg Leu Leu Thr Leu Val Val Gly Leu

1               5                   10                  15

Val Ile Leu Ser Gly Leu Ser Ile Leu Asp Phe Ser Ile Thr Ser Ala
            20                  25                  30

Ser Ala Gln Gln Ala Thr Asp Arg Ser Asn Ser Val Asn Tyr Ser Thr
        35                  40                  45

Asp Val Ile Tyr Gln Ile Val Thr Asp Arg Phe Tyr Asp Gly Asp Glu
    50                  55                  60

Ser Asn Asn Pro Ser Gly Glu Leu Tyr Ser Glu Asp Cys Lys Asn Leu
65                  70                  75                  80

Arg Lys Tyr Cys Gly Gly Asp Trp Gln Gly Ile Ile Asp Lys Ile Asp
                85                  90                  95

Asp Gly Tyr Leu Thr Asn Met Gly Val Thr Ala Leu Trp Ile Ser Pro
            100                 105                 110

Pro Val Glu Asn Ile Phe Glu Thr Ile Asp Asp Glu Phe Gly Thr Thr
        115                 120                 125

Ser Tyr His Gly Tyr Trp Ala Arg Asp Tyr Lys Lys Thr Asn Pro Phe
    130                 135                 140

Phe Gly Ser Thr Glu Asp Phe Glu Arg Leu Ile Glu Thr Ala His Ser
145                 150                 155                 160

His Asp Ile Lys Ile Val Ile Asp Leu Ala Pro Asn His Thr Ser Pro
                165                 170                 175

Ala Asp Phe Asp Asn Pro Asp Tyr Ala Glu Asn Gly Val Leu Tyr Asp
            180                 185                 190

Asp Gly Asn Tyr Leu Gly Ser Tyr Ser Asp Asp Ser Asp Leu Phe Leu
        195                 200                 205

Tyr Asn Gly Gly Thr Asp Phe Ser Asn Tyr Glu Asp Glu Ile Tyr Arg
    210                 215                 220

Asn Leu Phe Asp Leu Ala Ser Phe Asn His Ile Asn Ser Glu Leu Asn
225                 230                 235                 240

Asn Tyr Leu Glu Asp Ala Val Lys Lys Trp Leu Asp Leu Gly Ile Asp
                245                 250                 255

Gly Ile Arg Ile Asp Ala Val Ala His Met Pro Pro Gly Trp Lys Lys
            260                 265                 270

Ala Tyr Met Asp Thr Ile Tyr Asp His Arg Ala Val Phe Thr Phe Gly
        275                 280                 285

Glu Trp Phe Thr Gly Pro Ser Gly Asn Glu Asp Tyr Thr Lys Phe Ala
    290                 295                 300

Asn Asn Ser Gly Met Ser Val Leu Asp Phe Arg Phe Ala Gln Thr Thr
305                 310                 315                 320

Arg Asn Val Ile Gly Asn Asn Asn Gly Thr Met Tyr Asp Ile Glu Lys
                325                 330                 335

Met Leu Thr Asp Thr Glu Asn Asp Tyr Asp Arg Pro Gln Asp Gln Val
            340                 345                 350

Thr Phe Leu Asp Asn His Asp Met Ser Arg Phe Thr Asn Gly Gly Glu
        355                 360                 365

Ser Thr Arg Thr Thr Asp Ile Gly Leu Ala Leu Met Leu Thr Ser Arg
    370                 375                 380

Gly Val Pro Thr Ile Tyr Tyr Gly Thr Glu Gln Tyr Met Lys Gly Asp
385                 390                 395                 400

Gly Asp Pro Gly Ser Arg Gly Met Met Ala Ser Phe Asp Glu Asn Thr
                405                 410                 415

Asp Ala Tyr Lys Leu Ile Gln Lys Leu Ala Pro Leu Arg Lys Ser Asn
            420                 425                 430

Pro Ala Tyr Gly Tyr Gly Thr Thr Thr Glu Arg Trp Ile Asn Asp Asp
        435                 440                 445

Val Leu Ile Tyr Glu Arg His Phe Gly Glu Asn Tyr Ala Leu Ile Ala
    450                 455                 460

Ile Asn Arg Ser Leu Asn Thr Ser Tyr Asn Ile Gln Gly Leu Gln Thr
465                 470                 475                 480

Glu Met Pro Ser Asn Ser Tyr Asp Asp Val Leu Asp Gly Leu Leu Asp
                485                 490                 495

Gly Gln Ser Ile Val Val Asp Asn Lys Gly Gly Val Asn Glu Phe Gln
            500                 505                 510

Met Ser Pro Gly Glu Val Ser Val Trp Glu Phe Glu Ala Glu Asn Val
        515                 520                 525

Asp Lys Pro Ser Ile Gly Gln Val Gly Pro Ile Ile Gly Glu Ala Gly
    530                 535                 540

Arg Thr Val Thr Ile Ser Gly Glu Gly Phe Gly Ser Ser Gln Gly Thr
545                 550                 555                 560

Val His Phe Gly Ser Thr Ser Ala Glu Ile Leu Ser Trp Asn Asp Thr
                565                 570                 575

Ile Ile Thr Leu Thr Val Pro Asn Asn Glu Ala Gly Tyr His Asp Ile
            580                 585                 590

Thr Val Val Thr Glu Asp Glu Gln Val Ser Asn Ala Tyr Glu Phe Glu
        595                 600                 605

Val Leu Thr Ala Asp Gln Val Thr Val Arg Phe Ile Ile Asp Asn Ala
    610                 615                 620

Glu Thr Lys Leu Gly Glu Asn Val Phe Leu Val Gly Asn Val His Glu
625                 630                 635                 640

Leu Gly Asn Trp Asp Pro Glu Gln Ser Val Gly Arg Phe Phe Asn Gln
                645                 650                 655

Ile Val Tyr Gln Tyr Pro Thr Trp Tyr Tyr Asp Val Asn Val Pro Ala
            660                 665                 670

Asn Thr Asp Leu Glu Phe Lys Phe Ile Lys Ile Asp Gln Asp Asn Asn
        675                 680                 685

Val Ile Trp Gln Ser Gly Ala Asn Gln Thr Tyr Ser Ser Pro Glu Ser
    690                 695                 700

Gly Thr Gly Ile Ile Arg Val Asp Trp
705                 710

<210>  3
<211>  714
<212>  PRT
<213>  Panibacillus macerans

<400>  3

Met Lys Ser Arg Tyr Lys Arg Leu Thr Ser Leu Ala Leu Ser Leu Ser
1               5                   10                  15

Met Ala Leu Gly Ile Ser Leu Pro Ala Trp Ala Ser Pro Asp Thr Ser
            20                  25                  30

Val Asp Asn Lys Val Asn Phe Ser Thr Asp Val Ile Tyr Gln Ile Val
        35                  40                  45

Thr Asp Arg Phe Ala Asp Gly Asp Arg Thr Asn Asn Pro Ala Gly Asp
    50                  55                  60

Ala Phe Ser Gly Asp Arg Ser Asn Leu Lys Leu Tyr Phe Gly Gly Asp
65                  70                  75                  80

Trp Gln Gly Ile Ile Asp Lys Ile Asn Asp Gly Tyr Leu Thr Gly Met

                85                  90                  95

Gly Val Thr Ala Leu Trp Ile Ser Gln Pro Val Glu Asn Ile Thr Ser
            100                 105                 110

Val Ile Lys Tyr Ser Gly Val Asn Asn Thr Ser Tyr His Gly Tyr Trp
        115                 120                 125

Ala Arg Asp Phe Lys Gln Thr Asn Asp Ala Phe Gly Asp Phe Ala Asp
    130                 135                 140

Phe Gln Asn Leu Ile Asp Thr Ala His Ala His Asn Ile Lys Val Val
145                 150                 155                 160

Ile Asp Phe Ala Pro Asn His Thr Ser Pro Ala Asp Arg Asp Asn Pro
                165                 170                 175

Gly Phe Ala Glu Asn Gly Gly Met Tyr Asp Asn Gly Ser Leu Leu Gly
            180                 185                 190

Ala Tyr Ser Asn Asp Thr Ala Gly Leu Phe His His Asn Gly Gly Thr
        195                 200                 205

Asp Phe Ser Thr Ile Glu Asp Gly Ile Tyr Lys Asn Leu Tyr Asp Leu
    210                 215                 220

Ala Asp Ile Asn His Asn Asn Asn Ala Met Asp Ala Tyr Phe Lys Ser
225                 230                 235                 240

Ala Ile Asp Leu Trp Leu Gly Met Gly Val Asp Gly Ile Arg Phe Asp
                245                 250                 255

Ala Val Lys His Met Pro Phe Gly Trp Gln Lys Ser Phe Val Ser Ser
            260                 265                 270

Ile Tyr Gly Gly Asp His Pro Val Phe Thr Phe Gly Glu Trp Tyr Leu
        275                 280                 285

Gly Ala Asp Gln Thr Asp Gly Asp Asn Ile Lys Phe Ala Asn Glu Ser
    290                 295                 300

Gly Met Asn Leu Leu Asp Phe Glu Tyr Ala Gln Glu Val Arg Glu Val
305                 310                 315                 320

Phe Arg Asp Lys Thr Glu Thr Met Lys Asp Leu Tyr Glu Val Leu Ala
                325                 330                 335

Ser Thr Glu Ser Gln Tyr Asp Tyr Ile Asn Asn Met Val Thr Phe Ile
            340                 345                 350

Asp Asn His Asp Met Asp Arg Phe Gln Val Ala Gly Ser Gly Thr Arg
        355                 360                 365

Ala Thr Glu Gln Ala Leu Ala Leu Thr Leu Thr Ser Arg Gly Val Pro
    370                 375                 380

Ala Ile Tyr Tyr Gly Thr Glu Gln Tyr Met Thr Gly Asp Gly Asp Pro
385                 390                 395                 400

Asn Asn Arg Ala Met Met Thr Ser Phe Asn Thr Gly Thr Thr Ala Tyr
                405                 410                 415

Lys Val Ile Gln Ala Leu Ala Pro Leu Arg Lys Ser Asn Pro Ala Ile
            420                 425                 430

Ala Tyr Gly Thr Thr Thr Glu Arg Trp Val Asn Asn Asp Val Leu Ile
        435                 440                 445

Ile Glu Arg Lys Phe Gly Ser Ser Ala Ala Leu Val Ala Ile Asn Arg
    450                 455                 460

Asn Ser Ser Ala Ala Tyr Pro Ile Ser Gly Leu Leu Ser Ser Leu Pro
465                 470                 475                 480

Ala Gly Thr Tyr Ser Asp Val Leu Asn Gly Leu Leu Asn Gly Asn Ser
                485                 490                 495

Ile Thr Val Gly Ser Gly Gly Ala Val Thr Asn Phe Thr Leu Ala Ala
            500                 505                 510

Gly Gly Thr Ala Val Trp Gln Tyr Thr Ala Pro Glu Thr Ser Pro Ala
        515                 520                 525

Ile Gly Asn Val Gly Pro Thr Met Gly Gln Pro Gly Asn Ile Val Thr
    530                 535                 540

Ile Asp Gly Arg Gly Phe Gly Gly Thr Ala Gly Thr Val Tyr Phe Gly
545                 550                 555                 560

Thr Thr Ala Val Thr Gly Ser Gly Ile Val Ser Trp Glu Asp Thr Gln
                565                 570                 575

Ile Lys Ala Val Ile Pro Lys Val Ala Ala Gly Lys Thr Gly Val Ser
            580                 585                 590

Val Lys Thr Ser Ser Gly Thr Ala Ser Asn Thr Phe Lys Ser Phe Asn
        595                 600                 605

Val Leu Thr Gly Asp Gln Val Thr Val Arg Phe Leu Val Asn Gln Ala
    610                 615                 620

Asn Thr Asn Tyr Gly Thr Asn Val Tyr Leu Val Gly Asn Ala Ala Glu
625                 630                 635                 640

Leu Gly Ser Trp Asp Pro Asn Lys Ala Ile Gly Pro Met Tyr Asn Gln
                645                 650                 655

Val Ile Ala Lys Tyr Pro Ser Trp Tyr Tyr Asp Val Ser Val Pro Ala
            660                 665                 670

Gly Thr Lys Leu Asp Phe Lys Phe Ile Lys Lys Gly Gly Gly Thr Val
        675                 680                 685

Thr Trp Glu Gly Gly Gly Asn His Thr Tyr Thr Thr Pro Ala Ser Gly
    690                 695                 700

Val Gly Thr Val Thr Val Asp Trp Gln Asn
705                 710

<210>  4
<211>  713
<212>  PRT
<213>  Panibacillus macerans

<400>  4

Met Lys Lys Gln Val Lys Trp Leu Thr Ser Val Ser Met Ser Val Gly
1               5                   10                  15

Ile Ala Leu Gly Ala Ala Leu Pro Val Trp Ala Ser Pro Asp Thr Ser
            20                  25                  30

Val Asn Asn Lys Leu Asn Phe Ser Thr Asp Thr Val Tyr Gln Ile Val
        35                  40                  45

Thr Asp Arg Phe Val Asp Gly Asn Ser Ala Asn Asn Pro Thr Gly Ala
    50                  55                  60

Ala Phe Ser Ser Asp His Ser Asn Leu Lys Leu Tyr Phe Gly Gly Asp
65                  70                  75                  80

Trp Gln Gly Ile Thr Asn Lys Ile Asn Asp Gly Tyr Leu Thr Gly Met
                85                  90                  95

Gly Ile Thr Ala Leu Trp Ile Ser Gln Pro Val Glu Asn Ile Thr Ala
            100                 105                 110

Val Ile Asn Tyr Ser Gly Val Asn Asn Thr Ala Tyr His Gly Tyr Trp
        115                 120                 125

Pro Arg Asp Phe Lys Lys Thr Asn Ala Ala Phe Gly Ser Phe Thr Asp
    130                 135                 140

Phe Ser Asn Leu Ile Ala Ala Ala His Ser His Asn Ile Lys Val Val
145                 150                 155                 160

Met Asp Phe Ala Pro Asn His Thr Asn Pro Ala Ser Ser Thr Asp Pro

165                 170                 175

Ser Phe Ala Glu Asn Gly Ala Leu Tyr Asn Asn Gly Thr Leu Leu Gly
            180                 185                 190

Lys Tyr Ser Asn Asp Thr Ala Gly Leu Phe His His Asn Gly Gly Thr
        195                 200                 205

Asp Phe Ser Thr Thr Glu Ser Gly Ile Tyr Lys Asn Leu Tyr Asp Leu
    210                 215                 220

Ala Asp Ile Asn Gln Asn Asn Asn Thr Ile Asp Ser Tyr Leu Lys Glu
225                 230                 235                 240

Ser Ile Gln Leu Trp Leu Asn Leu Gly Val Asp Gly Ile Arg Phe Asp
                245                 250                 255

Ala Val Lys His Met Pro Gln Gly Trp Gln Lys Ser Tyr Val Ser Ser
            260                 265                 270

Ile Tyr Ser Ser Ala Asn Pro Val Phe Thr Phe Gly Glu Trp Phe Leu
        275                 280                 285

Gly Pro Asp Glu Met Thr Gln Asp Asn Ile Asn Phe Ala Asn Gln Ser
    290                 295                 300

Gly Met His Leu Leu Asp Phe Ala Phe Ala Gln Glu Ile Arg Glu Val
305                 310                 315                 320

Phe Arg Asp Lys Ser Glu Thr Met Thr Asp Leu Asn Ser Val Ile Ser
                325                 330                 335

Ser Thr Gly Ser Ser Tyr Asn Tyr Ile Asn Asn Met Val Thr Phe Ile
            340                 345                 350

Asp Asn His Asp Met Asp Arg Phe Gln Gln Ala Gly Ala Ser Thr Arg
        355                 360                 365

Pro Thr Glu Gln Ala Leu Ala Val Thr Leu Thr Ser Arg Gly Val Pro
    370                 375                 380

Ala Ile Tyr Tyr Gly Thr Glu Gln Tyr Met Thr Gly Asn Gly Asp Pro
385                 390                 395                 400

Asn Asn Arg Gly Met Met Thr Gly Phe Asp Thr Asn Lys Thr Ala Tyr
                405                 410                 415

Lys Val Ile Lys Ala Leu Ala Pro Leu Arg Lys Ser Asn Pro Ala Leu
            420                 425                 430

Ala Tyr Gly Ser Thr Thr Gln Arg Trp Val Asn Ser Asp Val Tyr Val
        435                 440                 445

Tyr Glu Arg Lys Phe Gly Ser Asn Val Ala Leu Val Ala Val Asn Arg
    450                 455                 460

Ser Ser Thr Thr Ala Tyr Pro Ile Ser Gly Ala Leu Thr Ala Leu Pro
465                 470                 475                 480

Asn Gly Thr Tyr Thr Asp Val Leu Gly Gly Leu Leu Asn Gly Asn Ser
                485                 490                 495

Ile Thr Val Asn Gly Gly Thr Val Ser Asn Phe Thr Leu Ala Ala Gly
            500                 505                 510

Gly Thr Ala Val Trp Gln Tyr Thr Thr Thr Glu Ser Ser Pro Ile Ile
        515                 520                 525

Gly Asn Val Gly Pro Thr Met Gly Lys Pro Gly Asn Thr Ile Thr Ile
    530                 535                 540

Asp Gly Arg Gly Phe Gly Thr Thr Lys Asn Lys Val Thr Phe Gly Thr
545                 550                 555                 560

Thr Ala Val Thr Gly Ala Asn Ile Val Ser Trp Glu Asp Thr Glu Ile
                565                 570                 575

Lys Val Lys Val Pro Asn Val Ala Ala Gly Asn Thr Ala Val Thr Val
            580                 585                 590

Thr Asn Ala Ala Gly Thr Thr Ser Ala Ala Phe Asn Asn Phe Asn Val
        595                 600                 605

Leu Thr Ala Asp Gln Val Thr Val Arg Phe Lys Val Asn Asn Ala Thr
    610                 615                 620

Thr Ala Leu Gly Gln Asn Val Tyr Leu Thr Gly Asn Val Ala Glu Leu
625                 630                 635                 640

Gly Asn Trp Thr Ala Ala Asn Ala Ile Gly Pro Met Tyr Asn Gln Val
                645                 650                 655

Glu Ala Ser Tyr Pro Thr Trp Tyr Phe Asp Val Ser Val Pro Ala Asn
            660                 665                 670

Thr Ala Leu Gln Phe Lys Phe Ile Lys Val Asn Gly Ser Thr Val Thr
        675                 680                 685

Trp Glu Gly Gly Asn Asn His Thr Phe Thr Ser Pro Ser Ser Gly Val
    690                 695                 700

Ala Thr Val Thr Val Asp Trp Gln Asn
705                 710

<210>  5
<211>  683
<212>  PRT
<213>  Thermoanaerobacterium thermosulfurigenes

<400>  5

Ala Ser Asp Thr Ala Val Ser Asn Val Val Asn Tyr Ser Thr Asp Val
1               5                   10                  15

Ile Tyr Gln Ile Val Thr Asp Arg Phe Val Asp Gly Asn Thr Ser Asn
            20                  25                  30

Asn Pro Thr Gly Asp Leu Tyr Asp Pro Thr His Thr Ser Leu Lys Lys
        35                  40                  45

Tyr Phe Gly Gly Asp Trp Gln Gly Ile Ile Asn Lys Ile Asn Asp Gly
    50                  55                  60

Tyr Leu Thr Gly Met Gly Val Thr Ala Ile Trp Ile Ser Gln Pro Val
65                  70                  75                  80

Glu Asn Ile Tyr Ala Val Leu Pro Asp Ser Thr Phe Gly Gly Ser Thr
                85                  90                  95

Ser Tyr His Gly Tyr Trp Ala Arg Asp Phe Lys Arg Thr Asn Pro Tyr
            100                 105                 110

Phe Gly Ser Phe Thr Asp Phe Gln Asn Leu Ile Asn Thr Ala His Ala
        115                 120                 125

His Asn Ile Lys Val Ile Ile Asp Phe Ala Pro Asn His Thr Ser Pro
    130                 135                 140

Ala Ser Glu Thr Asp Pro Thr Tyr Ala Glu Asn Gly Arg Leu Tyr Asp
145                 150                 155                 160

Asn Gly Thr Leu Leu Gly Gly Tyr Thr Asn Asp Thr Asn Gly Tyr Phe
                165                 170                 175

His His Tyr Gly Gly Thr Asp Phe Ser Ser Tyr Glu Asp Gly Ile Tyr
            180                 185                 190

Arg Asn Leu Phe Asp Leu Ala Asp Leu Asn Gln Gln Asn Ser Thr Ile
        195                 200                 205

Asp Ser Tyr Leu Lys Ser Ala Ile Lys Val Trp Leu Asp Met Gly Ile
    210                 215                 220

Asp Gly Ile Arg Leu Asp Ala Val Lys His Met Pro Phe Gly Trp Gln
225                 230                 235                 240

Lys Asn Phe Met Asp Ser Ile Leu Ser Tyr Arg Pro Val Phe Thr Phe

                245                 250                 255

Gly Glu Trp Phe Leu Gly Thr Asn Glu Ile Asp Val Asn Asn Thr Tyr
            260                 265                 270

Phe Ala Asn Glu Ser Gly Met Ser Leu Leu Asp Phe Arg Phe Ser Gln
        275                 280                 285

Lys Val Arg Gln Val Phe Arg Asp Asn Thr Asp Thr Met Tyr Gly Leu
    290                 295                 300

Asp Ser Met Ile Gln Ser Thr Ala Ser Asp Tyr Asn Phe Ile Asn Asp
305                 310                 315                 320

Met Val Thr Phe Ile Asp Asn His Asp Met Asp Arg Phe Tyr Asn Gly
                325                 330                 335

Gly Ser Thr Arg Pro Val Glu Gln Ala Leu Ala Phe Thr Leu Thr Ser
            340                 345                 350

Arg Gly Val Pro Ala Ile Tyr Tyr Gly Thr Glu Gln Tyr Met Thr Gly
        355                 360                 365

Asn Gly Asp Pro Tyr Asn Arg Ala Met Met Thr Ser Phe Asn Thr Ser
    370                 375                 380

Thr Thr Ala Tyr Asn Val Ile Lys Lys Leu Ala Pro Leu Arg Lys Ser
385                 390                 395                 400

Asn Pro Ala Ile Ala Tyr Gly Thr Thr Gln Gln Arg Trp Ile Asn Asn
                405                 410                 415

Asp Val Tyr Ile Tyr Glu Arg Lys Phe Gly Asn Asn Val Ala Leu Val
            420                 425                 430

Ala Ile Asn Arg Asn Leu Ser Thr Ser Tyr Asn Ile Thr Gly Leu Tyr
        435                 440                 445

Thr Ala Leu Pro Ala Gly Thr Tyr Thr Asp Val Leu Gly Gly Leu Leu
    450                 455                 460

Asn Gly Asn Ser Ile Ser Val Ala Ser Asp Gly Ser Val Thr Pro Phe
465                 470                 475                 480

Thr Leu Ser Ala Gly Glu Val Ala Val Trp Gln Tyr Val Ser Ser Ser
                485                 490                 495

Asn Ser Pro Leu Ile Gly His Val Gly Pro Thr Met Thr Lys Ala Gly
            500                 505                 510

Gln Thr Ile Thr Ile Asp Gly Arg Gly Phe Gly Thr Thr Ser Gly Gln
        515                 520                 525

Val Leu Phe Gly Ser Thr Ala Gly Thr Ile Val Ser Trp Asp Asp Thr
    530                 535                 540

Glu Val Lys Val Lys Val Pro Ser Val Thr Pro Gly Lys Tyr Asn Ile
545                 550                 555                 560

Ser Leu Lys Thr Ser Ser Gly Ala Thr Ser Asn Thr Tyr Asn Asn Ile
                565                 570                 575

Asn Ile Leu Thr Gly Asn Gln Ile Cys Val Arg Phe Val Val Asn Asn
            580                 585                 590

Ala Ser Thr Val Tyr Gly Glu Asn Val Tyr Leu Thr Gly Asn Val Ala
        595                 600                 605

Glu Leu Gly Asn Trp Asp Thr Ser Lys Ala Ile Gly Pro Met Phe Asn
    610                 615                 620

Gln Val Val Tyr Gln Tyr Pro Thr Trp Tyr Tyr Asp Val Ser Val Pro
625                 630                 635                 640

Ala Gly Thr Thr Ile Gln Phe Lys Phe Ile Lys Lys Asn Gly Asn Thr
                645                 650                 655

Ile Thr Trp Glu Gly Gly Ser Asn His Thr Tyr Thr Val Pro Ser Ser
            660                 665                 670

Ser Thr Gly Thr Val Ile Val Asn Trp Gln Gln
        675                 680

<210> 6
<211> 683
<212> PRT
<213> Thermoanaerobacter sp.

<400> 6

Ala Pro Asp Thr Ser Val Ser Asn Val Val Asn Tyr Ser Thr Asp Val
1               5                   10                  15

Ile Tyr Gln Ile Val Thr Asp Arg Phe Leu Asp Gly Asn Pro Ser Asn
            20                  25                  30

Asn Pro Thr Gly Asp Leu Tyr Asp Pro Thr His Thr Ser Leu Lys Lys
        35                  40                  45

Tyr Phe Gly Gly Asp Trp Gln Gly Ile Ile Asn Lys Ile Asn Asp Gly
    50                  55                  60

Tyr Leu Thr Gly Met Gly Ile Thr Ala Ile Trp Ile Ser Gln Pro Val
65                  70                  75                  80

Glu Asn Ile Tyr Ala Val Leu Pro Asp Ser Thr Phe Gly Gly Ser Thr
                85                  90                  95

Ser Tyr His Gly Tyr Trp Ala Arg Asp Phe Lys Lys Thr Asn Pro Phe
            100                 105                 110

Phe Gly Ser Phe Thr Asp Phe Gln Asn Leu Ile Ala Thr Ala His Ala
        115                 120                 125

His Asn Ile Lys Val Ile Ile Asp Phe Ala Pro Asn His Thr Ser Pro
    130                 135                 140

Ala Ser Glu Thr Asp Pro Thr Tyr Gly Glu Asn Gly Arg Leu Tyr Asp
145                 150                 155                 160

Asn Gly Val Leu Leu Gly Gly Tyr Thr Asn Asp Thr Asn Gly Tyr Phe
                165                 170                 175

His His Tyr Gly Gly Thr Asn Phe Ser Ser Tyr Glu Asp Gly Ile Tyr
            180                 185                 190

Arg Asn Leu Phe Asp Leu Ala Asp Leu Asp Gln Gln Asn Ser Thr Ile
        195                 200                 205

Asp Ser Tyr Leu Lys Ala Ala Ile Lys Leu Trp Leu Asp Met Gly Ile
    210                 215                 220

Asp Gly Ile Arg Met Asp Ala Val Lys His Met Ala Phe Gly Trp Gln
225                 230                 235                 240

Lys Asn Phe Met Asp Ser Ile Leu Ser Tyr Arg Pro Val Phe Thr Phe
                245                 250                 255

Gly Glu Trp Tyr Leu Gly Thr Asn Glu Val Asp Pro Asn Asn Thr Tyr
            260                 265                 270

Phe Ala Asn Glu Ser Gly Met Ser Leu Leu Asp Phe Arg Phe Ala Gln
        275                 280                 285

Lys Val Arg Gln Val Phe Arg Asp Asn Thr Asp Thr Met Tyr Gly Leu
    290                 295                 300

Asp Ser Met Ile Gln Ser Thr Ala Ala Asp Tyr Asn Phe Ile Asn Asp
305                 310                 315                 320

Met Val Thr Phe Ile Asp Asn His Asp Met Asp Arg Phe Tyr Thr Gly
                325                 330                 335

Gly Ser Thr Arg Pro Val Glu Gln Ala Leu Ala Phe Thr Leu Thr Ser
            340                 345                 350

Arg Gly Val Pro Ala Ile Tyr Tyr Gly Thr Glu Gln Tyr Met Thr Gly

        355                 360                 365

Asn Gly Asp Pro Tyr Asn Arg Ala Met Met Thr Ser Phe Asp Thr Thr
    370                 375                 380

Thr Thr Ala Tyr Asn Val Ile Lys Lys Leu Ala Pro Leu Arg Lys Ser
385                 390                 395                 400

Asn Pro Ala Ile Ala Tyr Gly Thr Gln Lys Gln Arg Trp Ile Asn Asn
                405                 410                 415

Asp Val Tyr Ile Tyr Glu Arg Gln Phe Gly Asn Asn Val Ala Leu Val
            420                 425                 430

Ala Ile Asn Arg Asn Leu Ser Thr Ser Tyr Tyr Ile Thr Gly Leu Tyr
        435                 440                 445

Thr Ala Leu Pro Ala Gly Thr Tyr Ser Asp Met Leu Gly Gly Leu Leu
    450                 455                 460

Asn Gly Ser Ser Ile Thr Val Ser Ser Asn Gly Ser Val Thr Pro Phe
465                 470                 475                 480

Thr Leu Ala Pro Gly Glu Val Ala Val Trp Gln Tyr Val Ser Thr Thr
                485                 490                 495

Asn Pro Pro Leu Ile Gly His Val Gly Pro Thr Met Thr Lys Ala Gly
            500                 505                 510

Gln Thr Ile Thr Ile Asp Gly Arg Gly Phe Gly Thr Thr Ala Gly Gln
        515                 520                 525

Val Leu Phe Gly Thr Thr Pro Ala Thr Ile Val Ser Trp Glu Asp Thr
    530                 535                 540

Glu Val Lys Val Lys Val Pro Ala Leu Thr Pro Gly Lys Tyr Asn Ile
545                 550                 555                 560

Thr Leu Lys Thr Ala Ser Gly Val Thr Ser Asn Ser Tyr Asn Asn Ile
                565                 570                 575

Asn Val Leu Thr Gly Asn Gln Val Cys Val Arg Phe Val Val Asn Asn
            580                 585                 590

Ala Thr Thr Val Trp Gly Glu Asn Val Tyr Leu Thr Gly Asn Val Ala
        595                 600                 605

Glu Leu Gly Asn Trp Asp Thr Ser Lys Ala Ile Gly Pro Met Phe Asn
    610                 615                 620

Gln Val Val Tyr Gln Tyr Pro Thr Trp Tyr Tyr Asp Val Ser Val Pro
625                 630                 635                 640

Ala Gly Thr Thr Ile Glu Phe Lys Phe Ile Lys Lys Asn Gly Ser Thr
                645                 650                 655

Val Thr Trp Glu Gly Gly Tyr Asn His Val Tyr Thr Thr Pro Thr Ser
            660                 665                 670

Gly Thr Ala Thr Val Ile Val Asp Trp Gln Pro
        675                 680

<210>  7
<211>  718
<212>  PRT
<213>  Bacillus circulans

<400>  7

Met Phe Gln Met Ala Lys Arg Ala Phe Leu Ser Thr Thr Leu Thr Leu
1               5                   10                  15

Gly Leu Leu Ala Gly Ser Ala Leu Pro Phe Leu Pro Ala Ser Ala Val
            20                  25                  30

Tyr Ala Asp Pro Asp Thr Ala Val Thr Asn Lys Gln Ser Phe Ser Thr
        35                  40                  45

Asp Val Ile Tyr Gln Val Phe Thr Asp Arg Phe Leu Asp Gly Asn Pro
    50                  55                  60

Ser Asn Asn Pro Thr Gly Ala Ala Tyr Asp Ala Thr Cys Ser Asn Leu
65                  70                  75                  80

Lys Leu Tyr Cys Gly Gly Asp Trp Gln Gly Leu Ile Asn Lys Ile Asn
                85                  90                  95

Asp Asn Tyr Phe Ser Asp Leu Gly Val Thr Ala Leu Trp Ile Ser Gln
            100                 105                 110

Pro Val Glu Asn Ile Phe Ala Thr Ile Asn Tyr Ser Gly Val Thr Asn
        115                 120                 125

Thr Ala Tyr His Gly Tyr Trp Ala Arg Asp Phe Lys Lys Thr Asn Pro
    130                 135                 140

Tyr Phe Gly Thr Met Ala Asp Phe Gln Asn Leu Ile Thr Thr Ala His
145                 150                 155                 160

Ala Lys Gly Ile Lys Ile Val Ile Asp Phe Ala Pro Asn His Thr Ser
                165                 170                 175

Pro Ala Met Glu Thr Asp Thr Ser Phe Ala Glu Asn Gly Arg Leu Tyr
            180                 185                 190

Asp Asn Gly Thr Leu Val Gly Gly Tyr Thr Asn Asp Thr Asn Gly Tyr
        195                 200                 205

Phe His His Asn Gly Gly Ser Asp Phe Ser Ser Leu Glu Asn Gly Ile
    210                 215                 220

Tyr Lys Asn Leu Tyr Asp Leu Ala Asp Phe Asn His Asn Asn Ala Thr
225                 230                 235                 240

Ile Asp Lys Tyr Phe Lys Asp Ala Ile Lys Leu Trp Leu Asp Met Gly
                245                 250                 255

Val Asp Gly Ile Arg Val Asp Ala Val Lys His Met Pro Leu Gly Trp
            260                 265                 270

Gln Lys Ser Trp Met Ser Ser Ile Tyr Ala His Lys Pro Val Phe Thr
        275                 280                 285

Phe Gly Glu Trp Phe Leu Gly Ser Ala Ala Ser Asp Ala Asp Asn Thr
    290                 295                 300

Asp Phe Ala Asn Lys Ser Gly Met Ser Leu Leu Asp Phe Arg Phe Asn
305                 310                 315                 320

Ser Ala Val Arg Asn Val Phe Arg Asp Asn Thr Ser Asn Met Tyr Ala
                325                 330                 335

Leu Asp Ser Met Ile Asn Ser Thr Ala Thr Asp Tyr Asn Gln Val Asn
            340                 345                 350

Asp Gln Val Thr Phe Ile Asp Asn His Asp Met Asp Arg Phe Lys Thr
        355                 360                 365

Ser Ala Val Asn Asn Arg Arg Leu Glu Gln Ala Leu Ala Phe Thr Leu
    370                 375                 380

Thr Ser Arg Gly Val Pro Ala Ile Tyr Tyr Gly Thr Glu Gln Tyr Leu
385                 390                 395                 400

Thr Gly Asn Gly Asp Pro Asp Asn Arg Ala Lys Met Pro Ser Phe Ser
                405                 410                 415

Lys Ser Thr Thr Ala Phe Asn Val Ile Ser Lys Leu Ala Pro Leu Arg
            420                 425                 430

Lys Ser Asn Pro Ala Ile Ala Tyr Gly Ser Thr Gln Gln Arg Trp Ile
        435                 440                 445

Asn Asn Asp Val Tyr Val Tyr Glu Arg Lys Phe Gly Lys Ser Val Ala
    450                 455                 460

Val Val Ala Val Asn Arg Asn Leu Ser Thr Ser Ala Ser Ile Thr Gly

465                 470                 475                 480

Leu Ser Thr Ser Leu Pro Thr Gly Ser Tyr Thr Asp Val Leu Gly Gly
                485                 490                 495

Val Leu Asn Gly Asn Asn Ile Thr Ser Thr Asn Gly Ser Ile Asn Asn
            500                 505                 510

Phe Thr Leu Ala Ala Gly Ala Thr Ala Val Trp Gln Tyr Thr Thr Ala
        515                 520                 525

Glu Thr Thr Pro Thr Ile Gly His Val Gly Pro Val Met Gly Lys Pro
    530                 535                 540

Gly Asn Val Val Thr Ile Asp Gly Arg Gly Phe Gly Ser Thr Lys Gly
545                 550                 555                 560

Thr Val Tyr Phe Gly Thr Thr Ala Val Thr Gly Ala Ala Ile Thr Ser
                565                 570                 575

Trp Glu Asp Thr Gln Ile Lys Val Thr Ile Pro Ser Val Ala Ala Gly
            580                 585                 590

Asn Tyr Ala Val Lys Val Ala Ala Ser Gly Val Asn Ser Asn Ala Tyr
        595                 600                 605

Asn Asn Phe Thr Ile Leu Thr Gly Asp Gln Val Thr Val Arg Phe Val
    610                 615                 620

Val Asn Asn Ala Ser Thr Thr Leu Gly Gln Asn Leu Tyr Leu Thr Gly
625                 630                 635                 640

Asn Val Ala Glu Leu Gly Asn Trp Ser Thr Gly Ser Thr Ala Ile Gly
                645                 650                 655

Pro Ala Phe Asn Gln Val Ile His Gln Tyr Pro Thr Trp Tyr Tyr Asp
            660                 665                 670

Val Ser Val Pro Ala Gly Lys Gln Leu Glu Phe Lys Phe Phe Lys Lys
        675                 680                 685

Asn Gly Ser Thr Ile Thr Trp Glu Ser Gly Ser Asn His Thr Phe Thr
    690                 695                 700

Thr Pro Ala Ser Gly Thr Ala Thr Val Thr Val Asn Trp Gln
705                 710                 715

<210>  8
<211>  718
<212>  PRT
<213>  Bacillus sp. 38-2

<400>  8

Met Phe Gln Met Ala Lys Arg Val Leu Leu Ser Thr Thr Leu Thr Phe
1               5                   10                  15

Ser Leu Leu Ala Gly Ser Ala Leu Pro Phe Leu Pro Ala Ser Ala Ile
            20                  25                  30

Tyr Ala Asp Ala Asp Thr Ala Val Thr Asn Lys Gln Asn Phe Ser Thr
        35                  40                  45

Asp Val Ile Tyr Gln Val Phe Thr Asp Arg Phe Leu Asp Gly Asn Pro
    50                  55                  60

Ser Asn Asn Pro Thr Gly Ala Ala Phe Asp Gly Thr Cys Ser Asn Leu
65                  70                  75                  80

Lys Leu Tyr Cys Gly Gly Asp Trp Gln Gly Leu Val Asn Lys Ile Asn
                85                  90                  95

Asp Asn Tyr Phe Ser Asp Leu Gly Val Thr Ala Leu Trp Ile Ser Gln
            100                 105                 110

Pro Val Glu Asn Ile Phe Ala Thr Ile Asn Tyr Ser Gly Val Thr Asn
        115                 120                 125

Thr Ala Tyr His Gly Tyr Trp Ala Arg Asp Phe Lys Lys Thr Asn Pro
    130                 135                 140

Tyr Phe Gly Thr Met Thr Asp Phe Gln Asn Leu Val Thr Thr Ala His
145                 150                 155                 160

Ala Lys Gly Ile Lys Ile Ile Ile Asp Phe Ala Pro Asn His Thr Ser
                165                 170                 175

Pro Ala Met Glu Thr Asp Thr Ser Phe Ala Glu Asn Gly Lys Leu Tyr
            180                 185                 190

Asp Asn Gly Asn Leu Val Gly Gly Tyr Thr Asn Asp Thr Asn Gly Tyr
        195                 200                 205

Phe His His Asn Gly Gly Ser Asp Phe Ser Thr Leu Glu Asn Gly Ile
    210                 215                 220

Tyr Lys Asn Leu Tyr Asp Leu Ala Asp Leu Asn His Asn Asn Ser Thr
225                 230                 235                 240

Ile Asp Thr Tyr Phe Lys Asp Ala Ile Lys Leu Trp Leu Asp Met Gly
                245                 250                 255

Val Asp Gly Ile Arg Val Asp Ala Val Lys His Met Pro Gln Gly Trp
            260                 265                 270

Gln Lys Asn Trp Met Ser Ser Ile Tyr Ala His Lys Pro Val Phe Thr
        275                 280                 285

Phe Gly Glu Trp Phe Leu Gly Ser Ala Ala Pro Asp Ala Asp Asn Thr
    290                 295                 300

Asp Phe Ala Asn Glu Ser Gly Met Ser Leu Leu Asp Phe Arg Phe Asn
305                 310                 315                 320

Ser Ala Val Arg Asn Val Phe Arg Asp Asn Thr Ser Asn Met Tyr Ala
                325                 330                 335

Leu Asp Ser Met Leu Thr Ala Thr Ala Ala Asp Tyr Asn Gln Val Asn
            340                 345                 350

Asp Gln Val Thr Phe Ile Asp Asn His Asp Met Asp Arg Phe Lys Thr
        355                 360                 365

Ser Ala Val Asn Asn Arg Arg Leu Glu Gln Ala Leu Ala Phe Thr Leu
    370                 375                 380

Thr Ser Arg Gly Val Pro Ala Ile Tyr Tyr Gly Thr Glu Gln Tyr Leu
385                 390                 395                 400

Thr Gly Asn Gly Asp Pro Asp Asn Arg Gly Lys Met Pro Ser Phe Ser
                405                 410                 415

Lys Ser Thr Thr Ala Phe Asn Val Ile Ser Lys Leu Ala Pro Leu Arg
            420                 425                 430

Lys Ser Asn Pro Ala Ile Ala Tyr Gly Ser Thr Gln Gln Arg Trp Ile
        435                 440                 445

Asn Asn Asp Val Tyr Ile Tyr Glu Arg Lys Phe Gly Lys Ser Val Ala
    450                 455                 460

Val Val Ala Val Asn Arg Asn Leu Thr Thr Pro Thr Ser Ile Thr Asn
465                 470                 475                 480

Leu Asn Thr Ser Leu Pro Ser Gly Thr Tyr Thr Asp Val Leu Gly Gly
                485                 490                 495

Val Leu Asn Gly Asn Asn Ile Thr Ser Ser Gly Gly Asn Ile Ser Ser
            500                 505                 510

Phe Thr Leu Ala Ala Gly Ala Thr Ala Val Trp Gln Tyr Thr Ala Ser
        515                 520                 525

Glu Thr Thr Pro Thr Ile Gly His Val Gly Pro Val Met Gly Lys Pro
    530                 535                 540

Gly Asn Val Val Thr Ile Asp Gly Arg Gly Phe Gly Ser Ala Lys Gly

545                 550                 555                 560

Thr Val Tyr Phe Gly Thr Thr Ala Val Thr Gly Ser Ala Ile Thr Ser
                565                 570                 575

Trp Glu Asp Thr Gln Ile Lys Val Thr Ile Pro Pro Val Ala Gly Gly
            580                 585                 590

Asp Tyr Ala Val Lys Val Ala Ala Asn Gly Val Asn Ser Asn Ala Tyr
        595                 600                 605

Asn Asp Phe Thr Ile Leu Ser Gly Asp Gln Val Ser Val Arg Phe Val
    610                 615                 620

Ile Asn Asn Ala Thr Thr Ala Leu Gly Glu Asn Ile Tyr Leu Thr Gly
625                 630                 635                 640

Asn Val Ser Glu Leu Gly Asn Trp Thr Thr Gly Ala Ala Ser Ile Gly
                645                 650                 655

Pro Ala Phe Asn Gln Val Ile His Ala Tyr Pro Thr Trp Tyr Tyr Asp
            660                 665                 670

Val Ser Val Pro Ala Gly Lys Gln Leu Glu Phe Lys Phe Phe Lys Lys
        675                 680                 685

Asn Gly Ala Thr Ile Thr Trp Glu Gly Gly Ser Asn His Thr Phe Thr
    690                 695                 700

Thr Pro Thr Ser Gly Thr Ala Thr Val Thr Ile Asn Trp Gln
705                 710                 715

<210>  9
<211>  713
<212>  PRT
<213>  Bacillus sp. 1011

<400>  9

Met Lys Arg Phe Met Lys Leu Thr Ala Val Trp Thr Leu Trp Leu Ser
1               5                   10                  15

Leu Thr Leu Gly Leu Leu Ser Pro Val His Ala Ala Pro Asp Thr Ser
            20                  25                  30

Val Ser Asn Lys Gln Asn Phe Ser Thr Asp Val Ile Tyr Gln Ile Phe
        35                  40                  45

Thr Asp Arg Phe Ser Asp Gly Asn Pro Ala Asn Asn Pro Thr Gly Ala
    50                  55                  60

Ala Phe Asp Gly Ser Cys Thr Asn Leu Arg Leu Tyr Cys Gly Gly Asp
65                  70                  75                  80

Trp Gln Gly Ile Ile Asn Lys Ile Asn Asp Gly Tyr Leu Thr Gly Met
                85                  90                  95

Gly Ile Thr Ala Ile Trp Ile Ser Gln Pro Val Glu Asn Ile Tyr Ser
            100                 105                 110

Val Ile Asn Tyr Ser Gly Val Asn Asn Thr Ala Tyr His Gly Tyr Trp
        115                 120                 125

Ala Arg Asp Phe Lys Lys Thr Asn Pro Ala Tyr Gly Thr Met Gln Asp
    130                 135                 140

Phe Lys Asn Leu Ile Asp Thr Ala His Ala His Asn Ile Lys Val Ile
145                 150                 155                 160

Ile Asp Phe Ala Pro Asn His Thr Ser Pro Ala Ser Ser Asp Asp Pro
                165                 170                 175

Ser Phe Ala Glu Asn Gly Arg Leu Tyr Asp Asn Gly Asn Leu Leu Gly
            180                 185                 190

Gly Tyr Thr Asn Asp Thr Gln Asn Leu Phe His His Tyr Gly Gly Thr
        195                 200                 205

Asp Phe Ser Thr Ile Glu Asn Gly Ile Tyr Lys Asn Leu Tyr Asp Leu
    210                 215                 220

Ala Asp Leu Asn His Asn Asn Ser Ser Val Asp Val Tyr Leu Lys Asp
225                 230                 235                 240

Ala Ile Lys Met Trp Leu Asp Leu Gly Val Asp Gly Ile Arg Val Asp
                245                 250                 255

Ala Val Lys His Met Pro Phe Gly Trp Gln Lys Ser Phe Met Ala Thr
            260                 265                 270

Ile Asn Asn Tyr Lys Pro Val Phe Thr Phe Gly Glu Trp Phe Leu Gly
        275                 280                 285

Val Asn Glu Ile Ser Pro Glu Tyr His Gln Phe Ala Asn Glu Ser Gly
    290                 295                 300

Met Ser Leu Leu Asp Phe Arg Phe Ala Gln Lys Ala Arg Gln Val Phe
305                 310                 315                 320

Arg Asp Asn Thr Asp Asn Met Tyr Gly Leu Lys Ala Met Leu Glu Gly
                325                 330                 335

Ser Glu Val Asp Tyr Ala Gln Val Asn Asp Gln Val Thr Phe Ile Asp
            340                 345                 350

Asn His Asp Met Glu Arg Phe His Thr Ser Asn Gly Asp Arg Arg Lys
        355                 360                 365

Leu Glu Gln Ala Leu Ala Phe Thr Leu Thr Ser Arg Gly Val Pro Ala
    370                 375                 380

Ile Tyr Tyr Gly Ser Glu Gln Tyr Met Ser Gly Gly Asn Asp Pro Asp
385                 390                 395                 400

Asn Arg Ala Arg Leu Pro Ser Phe Ser Thr Thr Thr Thr Ala Tyr Gln
                405                 410                 415

Val Ile Gln Lys Leu Ala Pro Leu Arg Lys Ser Asn Pro Ala Ile Ala
            420                 425                 430

Tyr Gly Ser Thr His Glu Arg Trp Ile Asn Asn Asp Val Ile Ile Tyr
        435                 440                 445

Glu Arg Lys Phe Gly Asn Asn Val Ala Val Val Ala Ile Asn Arg Asn
    450                 455                 460

Met Asn Thr Pro Ala Ser Ile Thr Gly Leu Val Thr Ser Leu Arg Arg
465                 470                 475                 480

Ala Ser Tyr Asn Asp Val Leu Gly Gly Ile Leu Asn Gly Asn Thr Leu
                485                 490                 495

Thr Val Gly Ala Gly Gly Ala Ala Ser Asn Phe Thr Leu Ala Pro Gly
            500                 505                 510

Gly Thr Ala Val Trp Gln Tyr Thr Thr Asp Ala Thr Thr Pro Ile Ile
        515                 520                 525

Gly Asn Val Gly Pro Met Met Ala Lys Pro Gly Val Thr Ile Thr Ile
    530                 535                 540

Asp Gly Arg Gly Phe Gly Ser Gly Lys Gly Thr Val Tyr Phe Gly Thr
545                 550                 555                 560

Thr Ala Val Thr Gly Ala Asp Ile Val Ala Trp Glu Asp Thr Gln Ile
                565                 570                 575

Gln Val Lys Ile Pro Ala Val Pro Gly Gly Ile Tyr Asp Ile Arg Val
            580                 585                 590

Ala Asn Ala Ala Gly Ala Ala Ser Asn Ile Tyr Asp Asn Phe Glu Val
        595                 600                 605

Leu Thr Gly Asp Gln Val Thr Val Arg Phe Val Ile Asn Asn Ala Thr
    610                 615                 620

Thr Ala Leu Gly Gln Asn Val Phe Leu Thr Gly Asn Val Ser Glu Leu

625                 630                 635                 640

Gly Asn Trp Asp Pro Asn Asn Ala Ile Gly Pro Met Tyr Asn Gln Val
                645                 650                 655

Val Tyr Gln Tyr Pro Thr Trp Tyr Tyr Asp Val Ser Val Pro Ala Gly
            660                 665                 670

Gln Thr Ile Glu Phe Lys Phe Leu Lys Lys Gln Gly Ser Thr Val Thr
        675                 680                 685

Trp Glu Gly Gly Ala Asn Arg Thr Phe Thr Thr Pro Thr Ser Gly Thr
    690                 695                 700

Ala Thr Val Asn Val Asn Trp Gln Pro
705                 710

<210>  10
<211>  712
<212>  PRT
<213>  Bacillus sp. 38-2

<400>  10

Met Lys Arg Phe Met Lys Leu Thr Ala Val Trp Thr Leu Trp Leu Ser
1               5                   10                  15

Leu Thr Leu Gly Leu Leu Ser Pro Val His Ala Ala Pro Asp Thr Ser
            20                  25                  30

Val Ser Asn Lys Gln Asn Phe Ser Thr Asp Val Ile Tyr Gln Ile Phe
        35                  40                  45

Thr Asp Arg Phe Ser Asp Gly Asn Pro Ala Asn Asn Pro Thr Gly Ala
    50                  55                  60

Ala Phe Asp Gly Ser Cys Thr Asn Leu Arg Leu Tyr Cys Gly Gly Asp
65                  70                  75                  80

Trp Gln Gly Ile Ile Asn Lys Ile Asn Asp Gly Tyr Leu Thr Gly Met
                85                  90                  95

Gly Ile Thr Ala Ile Trp Ile Ser Gln Pro Val Glu Asn Ile Tyr Ser
            100                 105                 110

Val Ile Asn Tyr Ser Gly Val His Asn Thr Ala Tyr His Gly Tyr Trp
        115                 120                 125

Ala Arg Asp Phe Lys Lys Thr Asn Pro Ala Tyr Gly Thr Met Gln Asp
    130                 135                 140

Phe Lys Asn Leu Ile Asp Thr Ala His Ala His Asn Ile Lys Val Ile
145                 150                 155                 160

Ile Asp Phe Ala Pro Asn His Thr Ser Pro Ala Ser Ser Asp Asp Pro
                165                 170                 175

Ser Phe Ala Glu Asn Gly Arg Leu Tyr Asp Asn Gly Asn Leu Leu Gly
            180                 185                 190

Gly Tyr Thr Asn Asp Thr Gln Asn Leu Phe His His Tyr Gly Gly Thr
        195                 200                 205

Asp Phe Ser Thr Ile Glu Asn Gly Ile Tyr Lys Asn Leu Tyr Asp Leu
    210                 215                 220

Ala Asp Leu Asn His Asn Asn Ser Ser Val Asp Val Tyr Leu Lys Asp
225                 230                 235                 240

Ala Ile Lys Met Trp Leu Asp Leu Gly Val Asp Gly Ile Arg Val Asp
                245                 250                 255

Ala Val Lys His Met Pro Phe Gly Trp Gln Lys Ser Phe Met Ser Thr
            260                 265                 270

Ile Asn Asn Tyr Lys Pro Val Phe Asn Phe Gly Glu Trp Phe Leu Gly
        275                 280                 285

Val Asn Glu Ile Ser Pro Glu Tyr His Gln Phe Ala Asn Glu Ser Gly
    290                 295                 300

Met Ser Leu Leu Asp Phe Pro Phe Ala Gln Lys Ala Arg Gln Val Phe
305                 310                 315                 320

Arg Asp Asn Thr Asp Asn Met Tyr Gly Leu Lys Ala Met Leu Glu Gly
                325                 330                 335

Ser Glu Val Asp Tyr Ala Gln Val Asn Asp Gln Val Thr Phe Ile Asp
            340                 345                 350

Asn His Asp Met Glu Arg Phe His Thr Ser Asn Gly Asp Arg Arg Lys
        355                 360                 365

Leu Glu Gln Ala Leu Ala Phe Thr Leu Thr Ser Arg Gly Val Pro Ala
    370                 375                 380

Ile Tyr Tyr Gly Ser Glu Gln Tyr Met Ser Gly Gly Asn Asp Pro Asp
385                 390                 395                 400

Asn Arg Ala Arg Ile Pro Ser Phe Ser Thr Thr Thr Thr Ala Tyr Gln
                405                 410                 415

Val Ile Gln Lys Leu Ala Pro Leu Arg Lys Ser Asn Pro Ala Ile Ala
            420                 425                 430

Tyr Gly Ser Thr Gln Glu Arg Trp Ile Asn Asn Asp Val Ile Ile Tyr
        435                 440                 445

Glu Arg Lys Phe Gly Asn Asn Val Ala Val Val Ala Ile Asn Arg Asn
    450                 455                 460

Met Asn Thr Pro Ala Ser Ile Thr Gly Leu Val Thr Ser Leu Pro Gln
465                 470                 475                 480

Gly Ser Tyr Asn Asp Val Leu Gly Gly Ile Leu Asn Gly Asn Thr Leu
                485                 490                 495

Thr Val Gly Ala Gly Gly Ala Ala Ser Asn Phe Thr Leu Ala Pro Gly
            500                 505                 510

Gly Thr Ala Val Trp Gln Tyr Thr Thr Asp Ala Thr Ala Pro Ile Asn
        515                 520                 525

Gly Asn Val Gly Pro Met Met Ala Lys Ala Gly Val Thr Ile Thr Ile
    530                 535                 540

Asp Gly Arg Ala Ser Ala Arg Gln Gly Thr Val Tyr Phe Gly Thr Thr
545                 550                 555                 560

Ala Val Thr Gly Ala Asp Ile Val Ala Trp Glu Asp Thr Gln Ile Gln
                565                 570                 575

Val Lys Ile Leu Arg Val Pro Gly Gly Ile Tyr Asp Ile Arg Val Ala
            580                 585                 590

Asn Ala Ala Gly Ala Ala Ser Asn Ile Tyr Asp Asn Phe Glu Val Leu
        595                 600                 605

Thr Gly Asp Gln Val Thr Val Arg Phe Val Ile Asn Asn Ala Thr Thr
    610                 615                 620

Ala Leu Gly Gln Asn Val Phe Leu Thr Gly Asn Val Ser Glu Leu Gly
625                 630                 635                 640

Asn Trp Asp Pro Asn Asn Ala Ile Gly Pro Met Tyr Asn Gln Val Val
                645                 650                 655

Tyr Gln Tyr Pro Thr Trp Tyr Tyr Asp Val Ser Val Pro Ala Gly Gln
            660                 665                 670

Thr Ile Glu Phe Lys Phe Leu Lys Lys Gln Gly Ser Thr Val Thr Trp
        675                 680                 685

Glu Gly Gly Ala Asn Arg Thr Phe Thr Thr Pro Thr Ser Gly Thr Ala
    690                 695                 700

Thr Val Asn Val Asn Trp Gln Pro

705                 710

<210>  11
<211>  713
<212>  PRT
<213>  Bacillus circulans

<400>  11

Met Lys Lys Phe Leu Lys Ser Thr Ala Ala Leu Ala Leu Gly Leu Ser
1               5                   10                  15

Leu Thr Phe Gly Leu Phe Ser Pro Ala Gln Ala Ala Pro Asp Thr Ser
            20                  25                  30

Val Ser Asn Lys Gln Asn Phe Ser Thr Asp Val Ile Tyr Gln Ile Phe
        35                  40                  45

Thr Asp Arg Phe Ser Asp Gly Asn Pro Ala Asn Asn Pro Thr Gly Ala
    50                  55                  60

Ala Phe Asp Gly Thr Cys Thr Asn Leu Arg Leu Tyr Cys Gly Gly Asp
65                  70                  75                  80

Trp Gln Gly Ile Ile Asn Lys Ile Asn Asp Gly Tyr Leu Thr Gly Met
                85                  90                  95

Gly Val Thr Ala Ile Trp Ile Ser Gln Pro Val Glu Asn Ile Tyr Ser
            100                 105                 110

Ile Ile Asn Tyr Ser Gly Val Asn Asn Thr Ala Tyr His Gly Tyr Trp
        115                 120                 125

Ala Arg Asp Phe Lys Lys Thr Asn Pro Ala Tyr Gly Thr Ile Ala Asp
    130                 135                 140

Phe Gln Asn Leu Ile Ala Ala Ala His Ala Lys Asn Ile Lys Val Ile
145                 150                 155                 160

Ile Asp Phe Ala Pro Asn His Thr Ser Pro Ala Ser Ser Asp Gln Pro
                165                 170                 175

Ser Phe Ala Glu Asn Gly Arg Leu Tyr Asp Asn Gly Thr Leu Leu Gly
            180                 185                 190

Gly Tyr Thr Asn Asp Thr Gln Asn Leu Phe His His Asn Gly Gly Thr
        195                 200                 205

Asp Phe Ser Thr Thr Glu Asn Gly Ile Tyr Lys Asn Leu Tyr Asp Leu
    210                 215                 220

Ala Asp Leu Asn His Asn Asn Ser Thr Val Asp Val Tyr Leu Lys Asp
225                 230                 235                 240

Ala Ile Lys Met Trp Leu Asp Leu Gly Ile Asp Gly Ile Arg Met Asp
                245                 250                 255

Ala Val Lys His Met Pro Phe Gly Trp Gln Lys Ser Phe Met Ala Ala
            260                 265                 270

Val Asn Asn Tyr Lys Pro Val Phe Thr Phe Gly Glu Trp Phe Leu Gly
        275                 280                 285

Val Asn Glu Val Ser Pro Glu Asn His Lys Phe Ala Asn Glu Ser Gly
    290                 295                 300

Met Ser Leu Leu Asp Phe Arg Phe Ala Gln Lys Val Arg Gln Val Phe
305                 310                 315                 320

Arg Asp Asn Thr Asp Asn Met Tyr Gly Leu Lys Ala Met Leu Glu Gly
                325                 330                 335

Ser Ala Ala Asp Tyr Ala Gln Val Asp Asp Gln Val Thr Phe Ile Asp
            340                 345                 350

Asn His Asp Met Glu Arg Phe His Ala Ser Asn Ala Asn Arg Arg Lys
        355                 360                 365

Leu Glu Gln Ala Leu Ala Phe Thr Leu Thr Ser Arg Gly Val Pro Ala
    370                 375                 380

Ile Tyr Tyr Gly Thr Glu Gln Tyr Met Ser Gly Gly Thr Asp Pro Asp
385                 390                 395                 400

Asn Arg Ala Arg Ile Pro Ser Phe Ser Thr Ser Thr Thr Ala Tyr Gln
                405                 410                 415

Val Ile Gln Lys Leu Ala Pro Leu Arg Lys Cys Asn Pro Ala Ile Ala
            420                 425                 430

Tyr Gly Ser Thr Gln Glu Arg Trp Ile Asn Asn Asp Val Leu Ile Tyr
        435                 440                 445

Glu Arg Lys Phe Gly Ser Asn Val Ala Val Val Ala Val Asn Arg Asn
    450                 455                 460

Leu Asn Ala Pro Ala Ser Ile Ser Gly Leu Val Thr Ser Leu Pro Gln
465                 470                 475                 480

Gly Ser Tyr Asn Asp Val Leu Gly Gly Leu Leu Asn Gly Asn Thr Leu
                485                 490                 495

Ser Val Gly Ser Gly Gly Ala Ala Ser Asn Phe Thr Leu Ala Ala Gly
            500                 505                 510

Gly Thr Ala Val Trp Gln Tyr Thr Ala Ala Thr Ala Thr Pro Thr Ile
        515                 520                 525

Gly His Val Gly Pro Met Met Ala Lys Pro Gly Val Thr Ile Thr Ile
    530                 535                 540

Asp Gly Arg Gly Phe Gly Ser Ser Lys Gly Thr Val Tyr Phe Gly Thr
545                 550                 555                 560

Thr Ala Val Ser Gly Ala Asp Ile Thr Ser Trp Glu Asp Thr Gln Ile
                565                 570                 575

Lys Val Lys Ile Pro Ala Val Ala Gly Gly Asn Tyr Asn Ile Lys Val
            580                 585                 590

Ala Asn Ala Ala Gly Thr Ala Ser Asn Val Tyr Asp Asn Phe Glu Val
        595                 600                 605

Leu Ser Gly Asp Gln Val Ser Val Arg Phe Val Val Asn Asn Ala Thr
    610                 615                 620

Thr Ala Leu Gly Gln Asn Val Tyr Leu Thr Gly Ser Val Ser Glu Leu
625                 630                 635                 640

Gly Asn Trp Asp Pro Ala Lys Ala Ile Gly Pro Met Tyr Asn Gln Val
                645                 650                 655

Val Tyr Gln Tyr Pro Asn Trp Tyr Tyr Asp Val Ser Val Pro Ala Gly
            660                 665                 670

Lys Thr Ile Glu Phe Lys Phe Leu Lys Lys Gln Gly Ser Thr Val Thr
        675                 680                 685

Trp Glu Gly Gly Ser Asn His Thr Phe Thr Ala Pro Ser Ser Gly Thr
    690                 695                 700

Ala Thr Ile Asn Val Asn Trp Gln Pro
705                 710

<210>  12
<211>  686
<212>  PRT
<213>  Bacillus sp.

<400>  12

Ala Pro Asp Thr Ser Val Ser Asn Lys Gln Asn Phe Ser Thr Asp Val
1               5                   10                  15

Ile Tyr Gln Ile Phe Thr Asp Arg Phe Ser Asp Gly Asn Pro Ala Asn
            20                  25                  30

Asn Pro Thr Gly Ala Ala Phe Asp Gly Ser Cys Thr Asn Leu Arg Leu
        35                  40                  45

Tyr Cys Gly Gly Asp Trp Gln Gly Ile Ile Asn Lys Ile Asn Asp Gly
    50                  55                  60

Tyr Leu Thr Gly Met Gly Ile Thr Ala Ile Trp Ile Ser Gln Pro Val
65                  70                  75                  80

Glu Asn Ile Tyr Ser Val Ile Asn Tyr Ser Gly Val Asn Asn Thr Ala
                85                  90                  95

Tyr His Gly Tyr Trp Ala Arg Asp Phe Lys Lys Thr Asn Pro Ala Tyr
            100                 105                 110

Gly Thr Met Gln Asp Phe Lys Asn Leu Ile Asp Thr Ala His Ala His
        115                 120                 125

Asn Ile Lys Val Ile Ile Asp Phe Ala Pro Asn His Thr Ser Pro Ala
    130                 135                 140

Ser Ser Asp Asp Pro Ser Phe Ala Glu Asn Gly Arg Leu Tyr Asp Asn
145                 150                 155                 160

Gly Asn Leu Leu Gly Gly Tyr Thr Asn Asp Thr Gln Asn Leu Phe His
                165                 170                 175

His Tyr Gly Gly Thr Asp Phe Ser Thr Ile Glu Asn Gly Ile Tyr Lys
            180                 185                 190

Asn Leu Tyr Asp Leu Ala Asp Leu Asn His Asn Asn Ser Ser Val Asp
        195                 200                 205

Val Tyr Leu Lys Asp Ala Ile Lys Met Trp Leu Asp Leu Gly Val Asp
    210                 215                 220

Gly Ile Arg Val Asp Ala Val Lys His Met Pro Phe Gly Trp Gln Lys
225                 230                 235                 240

Ser Phe Met Ser Thr Ile Asn Asn Tyr Lys Pro Val Phe Thr Phe Gly
                245                 250                 255

Glu Trp Phe Leu Gly Val Asn Glu Ile Ser Pro Glu Tyr His Gln Phe
            260                 265                 270

Ala Asn Glu Ser Gly Met Ser Leu Leu Asp Phe Arg Phe Ala Gln Lys
        275                 280                 285

Ala Arg Gln Val Phe Arg Asp Asn Thr Asp Asn Met Tyr Gly Leu Lys
    290                 295                 300

Ala Met Leu Glu Gly Ser Glu Val Asp Tyr Ala Gln Val Asn Asp Gln
305                 310                 315                 320

Val Thr Phe Ile Asp Asn His Asp Met Glu Arg Phe His Thr Ser Asn
                325                 330                 335

Gly Asp Arg Arg Lys Leu Glu Gln Ala Leu Ala Phe Thr Leu Thr Ser
            340                 345                 350

Arg Gly Val Pro Ala Ile Tyr Tyr Gly Ser Glu Gln Tyr Met Ser Gly
        355                 360                 365

Gly Asn Asp Pro Asp Asn Arg Ala Arg Ile Pro Ser Phe Ser Thr Thr
    370                 375                 380

Thr Thr Ala Tyr Gln Val Ile Gln Lys Leu Ala Pro Leu Arg Lys Ser
385                 390                 395                 400

Asn Pro Ala Ile Ala Tyr Gly Ser Thr Gln Glu Arg Trp Ile Asn Asn
                405                 410                 415

Asp Val Ile Ile Tyr Glu Arg Lys Phe Gly Asn Asn Val Ala Val Val
            420                 425                 430

Ala Ile Asn Arg Asn Met Asn Thr Pro Ala Ser Ile Thr Gly Leu Val
        435                 440                 445

Thr Ser Leu Pro Gln Gly Ser Tyr Asn Asp Val Leu Gly Gly Ile Leu
    450                 455                 460

Asn Gly Asn Thr Leu Thr Val Gly Ala Gly Gly Ala Ala Ser Asn Phe
465                 470                 475                 480

Thr Leu Ala Pro Gly Gly Thr Ala Val Trp Gln Tyr Thr Thr Asp Ala
                485                 490                 495

Thr Ala Pro Ile Ile Gly Asn Val Gly Pro Met Met Ala Lys Pro Gly
            500                 505                 510

Val Thr Ile Thr Ile Asp Gly Arg Gly Phe Gly Ser Gly Lys Gly Thr
        515                 520                 525

Val Tyr Phe Gly Thr Thr Ala Val Thr Gly Ala Asp Ile Val Ala Trp
    530                 535                 540

Glu Asp Thr Gln Ile Gln Val Lys Ile Pro Ala Val Pro Gly Gly Ile
545                 550                 555                 560

Tyr Asp Ile Arg Val Ala Asn Ala Ala Gly Ala Ala Ser Asn Ile Tyr
                565                 570                 575

Asp Asn Phe Glu Val Leu Thr Gly Asp Gln Val Thr Val Arg Phe Val
            580                 585                 590

Ile Asn Asn Ala Thr Thr Ala Leu Gly Gln Asn Val Phe Leu Thr Gly
        595                 600                 605

Asn Val Ser Glu Leu Gly Asn Trp Asp Pro Asn Asn Ala Ile Gly Pro
    610                 615                 620

Met Tyr Asn Gln Val Val Tyr Gln Tyr Pro Thr Trp Tyr Tyr Asp Val
625                 630                 635                 640

Ser Val Pro Ala Gly Gln Thr Ile Glu Phe Lys Phe Leu Lys Lys Gln
                645                 650                 655

Gly Ser Thr Val Thr Trp Glu Gly Gly Ala Asn Arg Thr Phe Thr Thr
            660                 665                 670

Pro Thr Ser Gly Thr Ala Thr Met Asn Val Asn Trp Gln Pro
        675                 680                 685

<210>  13
<211>  704
<212>  PRT
<213>  Bacillus ohbensis

<400>  13

Met Lys Asn Leu Thr Val Leu Leu Lys Thr Ile Pro Leu Ala Leu Leu
1               5                   10                  15

Leu Phe Ile Leu Leu Ser Leu Pro Thr Ala Ala Gln Ala Asp Val Thr
            20                  25                  30

Asn Lys Val Asn Tyr Thr Arg Asp Val Ile Tyr Gln Ile Val Thr Asp
        35                  40                  45

Arg Phe Ser Asp Gly Asp Pro Ser Asn Asn Pro Thr Gly Ala Ile Tyr
    50                  55                  60

Ser Gln Asp Cys Ser Asp Leu His Lys Tyr Cys Gly Gly Asp Trp Gln
65                  70                  75                  80

Gly Ile Ile Asp Lys Ile Asn Asp Gly Tyr Leu Thr Asp Leu Gly Ile
                85                  90                  95

Thr Ala Ile Trp Ile Ser Gln Pro Val Glu Asn Val Tyr Ala Leu His
            100                 105                 110

Pro Ser Gly Tyr Thr Ser Tyr His Gly Tyr Trp Ala Arg Asp Tyr Lys
        115                 120                 125

Arg Thr Asn Pro Phe Tyr Gly Asp Phe Ser Asp Phe Asp Arg Leu Met
    130                 135                 140

Asp Thr Ala His Ser Asn Gly Ile Lys Val Ile Met Asp Phe Thr Pro
145                 150                 155                 160

Asn His Ser Ser Pro Ala Leu Glu Thr Asp Pro Ser Tyr Ala Glu Asn
                165                 170                 175

Gly Ala Val Tyr Asn Asp Gly Val Leu Ile Gly Asn Tyr Ser Asn Asp
            180                 185                 190

Pro Asn Asn Leu Phe His His Asn Gly Gly Thr Asp Phe Ser Ser Tyr
        195                 200                 205

Glu Asp Ser Ile Tyr Arg Asn Leu Tyr Asp Leu Ala Asp Tyr Asp Leu
    210                 215                 220

Asn Asn Thr Val Met Asp Gln Tyr Leu Lys Glu Ser Ile Lys Leu Trp
225                 230                 235                 240

Leu Asp Lys Gly Ile Asp Gly Ile Arg Val Asp Ala Val Lys His Met
                245                 250                 255

Ser Glu Gly Trp Gln Thr Ser Leu Met Ser Asp Ile Tyr Ala His Glu
            260                 265                 270

Pro Val Phe Thr Phe Gly Glu Trp Phe Leu Gly Ser Gly Glu Val Asp
        275                 280                 285

Pro Gln Asn His His Phe Ala Asn Glu Ser Gly Met Ser Leu Leu Asp
    290                 295                 300

Phe Gln Phe Gly Gln Thr Ile Arg Asp Val Leu Met Asp Gly Ser Ser
305                 310                 315                 320

Asn Trp Tyr Asp Phe Asn Glu Met Ile Ala Ser Thr Glu Glu Asp Tyr
                325                 330                 335

Asp Glu Val Ile Asp Gln Val Thr Phe Ile Asp Asn His Asp Met Ser
            340                 345                 350

Arg Phe Ser Phe Glu Gln Ser Ser Asn Arg His Thr Asp Ile Ala Leu
        355                 360                 365

Ala Val Leu Leu Thr Ser Arg Gly Val Pro Thr Ile Tyr Tyr Gly Thr
    370                 375                 380

Glu Gln Tyr Leu Thr Gly Gly Asn Asp Pro Glu Asn Arg Lys Pro Met
385                 390                 395                 400

Ser Asp Phe Asp Arg Thr Thr Asn Ser Tyr Gln Ile Ile Ser Thr Leu
                405                 410                 415

Ala Ser Leu Arg Gln Asn Asn Pro Ala Leu Gly Tyr Gly Asn Thr Ser
            420                 425                 430

Glu Arg Trp Ile Asn Ser Asp Val Tyr Ile Tyr Glu Arg Ser Phe Gly
        435                 440                 445

Asp Ser Val Val Leu Thr Ala Val Asn Ser Gly Asp Thr Ser Tyr Thr
    450                 455                 460

Ile Asn Asn Leu Asn Thr Ser Leu Pro Gln Gly Gln Tyr Thr Asp Glu
465                 470                 475                 480

Leu Gln Gln Leu Leu Asp Gly Asn Glu Ile Thr Val Asn Ser Asn Gly
                485                 490                 495

Ala Val Asp Ser Phe Gln Leu Ser Ala Asn Gly Val Ser Val Trp Gln
            500                 505                 510

Ile Thr Glu Glu His Ala Ser Pro Leu Ile Gly His Val Gly Pro Met
        515                 520                 525

Met Gly Lys His Gly Asn Thr Val Thr Ile Thr Gly Glu Gly Phe Gly
    530                 535                 540

Asp Asn Glu Gly Ser Val Leu Phe Asp Ser Asp Phe Ser Asp Val Leu
545                 550                 555                 560

Ser Trp Ser Asp Thr Lys Ile Glu Val Ser Val Pro Asp Val Thr Ala
                565                 570                 575

Gly His Tyr Asp Ile Ser Val Val Asn Ala Gly Asp Ser Gln Ser Pro
            580                 585                 590

Thr Tyr Asp Lys Phe Glu Val Leu Thr Gly Asp Gln Val Ser Ile Arg
        595                 600                 605

Phe Ala Val Asn Asn Ala Thr Thr Ser Leu Gly Thr Asn Leu Tyr Met
    610                 615                 620

Val Gly Asn Val Asn Glu Leu Gly Asn Trp Asp Pro Asp Gln Ala Ile
625                 630                 635                 640

Gly Pro Met Phe Asn Gln Val Met Tyr Gln Tyr Pro Thr Trp Tyr Tyr
                645                 650                 655

Asp Ile Ser Val Pro Ala Glu Glu Asn Leu Glu Tyr Lys Phe Ile Lys
            660                 665                 670

Lys Asp Ser Ser Gly Asn Val Val Trp Glu Ser Gly Asn Asn His Thr
        675                 680                 685

Tyr Thr Thr Pro Ala Thr Gly Thr Asp Thr Val Leu Val Asp Trp Gln
    690                 695                 700

<210>  14
<211>  703
<212>  PRT
<213>  Bacillus sp. 1-1

<400>  14

Met Asn Asp Leu Asn Asp Phe Leu Lys Thr Ile Leu Leu Ser Phe Ile
1               5                   10                  15

Phe Phe Leu Leu Leu Ser Leu Pro Thr Val Ala Glu Ala Asp Val Thr
            20                  25                  30

Asn Lys Val Asn Tyr Ser Lys Asp Val Ile Tyr Gln Ile Val Thr Asp
        35                  40                  45

Arg Phe Ser Asp Gly Asn Pro Gly Asn Asn Pro Ser Gly Ala Ile Phe
    50                  55                  60

Ser Gln Asn Cys Ile Asp Leu His Lys Tyr Cys Gly Gly Asp Trp Gln
65                  70                  75                  80

Gly Ile Ile Asp Lys Ile Asn Asp Gly Tyr Leu Thr Asp Leu Gly Ile
                85                  90                  95

Thr Ala Leu Trp Ile Ser Gln Pro Val Glu Asn Val Tyr Ala Leu His
            100                 105                 110

Pro Ser Gly Tyr Thr Ser Tyr His Gly Tyr Trp Ala Arg Asp Tyr Lys
        115                 120                 125

Lys Thr Asn Pro Tyr Tyr Gly Asn Phe Asp Asp Phe Asp Arg Leu Met
    130                 135                 140

Ser Thr Ala His Ser Asn Gly Ile Lys Val Ile Met Asp Phe Thr Pro
145                 150                 155                 160

Asn His Ser Ser Pro Ala Leu Glu Thr Asn Pro Asn Tyr Val Glu Asn
                165                 170                 175

Gly Ala Ile Tyr Asp Asn Gly Ala Leu Leu Gly Asn Tyr Ser Asn Asp
            180                 185                 190

Gln Gln Asn Leu Phe His His Asn Gly Gly Thr Asp Phe Ser Ser Tyr
        195                 200                 205

Glu Asp Ser Ile Tyr Arg Asn Leu Tyr Asp Leu Ala Asp Tyr Asp Leu
    210                 215                 220

Asn Asn Thr Val Met Asp Gln Tyr Leu Lys Glu Ser Ile Lys Phe Trp
225                 230                 235                 240

Leu Asp Lys Gly Ile Asp Gly Ile Arg Val Asp Ala Val Lys His Met
                245                 250                 255

Ser Glu Gly Trp Gln Thr Ser Leu Met Ser Glu Ile Tyr Ser His Lys
            260                 265                 270

Pro Val Phe Thr Phe Gly Glu Trp Phe Leu Gly Ser Gly Glu Val Asp
        275                 280                 285

Pro Gln Asn His His Phe Ala Asn Glu Ser Gly Met Ser Leu Leu Asp
    290                 295                 300

Phe Gln Phe Gly Gln Thr Ile Arg Asn Val Leu Lys Asp Arg Thr Ser
305                 310                 315                 320

Asn Trp Tyr Asp Phe Asn Glu Met Ile Thr Ser Thr Glu Lys Glu Tyr
                325                 330                 335

Asn Glu Val Ile Asp Gln Val Thr Phe Ile Asp Asn His Asp Met Ser
            340                 345                 350

Arg Phe Ser Val Gly Ser Ser Ser Asn Arg Gln Thr Asp Met Ala Leu
        355                 360                 365

Ala Val Leu Leu Thr Ser Arg Gly Val Pro Thr Ile Tyr Tyr Gly Thr
    370                 375                 380

Glu Gln Tyr Val Thr Gly Gly Asn Asp Pro Glu Asn Arg Lys Pro Leu
385                 390                 395                 400

Lys Thr Phe Asp Arg Ser Thr Asn Ser Tyr Gln Ile Ile Ser Lys Leu
                405                 410                 415

Ala Ser Leu Arg Gln Thr Asn Ser Ala Leu Gly Tyr Gly Thr Thr Thr
            420                 425                 430

Glu Arg Trp Leu Asn Glu Asp Ile Tyr Ile Tyr Glu Arg Thr Phe Gly
        435                 440                 445

Asn Ser Ile Val Leu Thr Ala Val Asn Ser Ser Asn Ser Asn Gln Thr
    450                 455                 460

Ile Thr Asn Leu Asn Thr Ser Leu Pro Gln Gly Asn Tyr Thr Asp Glu
465                 470                 475                 480

Leu Gln Gln Arg Leu Asp Gly Asn Thr Ile Thr Val Asn Ala Asn Gly
                485                 490                 495

Ala Val Asn Ser Phe Gln Leu Arg Ala Asn Ser Val Ala Val Trp Gln
            500                 505                 510

Val Ser Asn Pro Ser Thr Ser Pro Leu Ile Gly Gln Val Gly Pro Met
        515                 520                 525

Met Gly Lys Ala Gly Asn Thr Ile Thr Val Ser Gly Glu Gly Phe Gly
    530                 535                 540

Asp Glu Arg Gly Ser Val Leu Phe Asp Ser Thr Ser Ser Glu Ile Ile
545                 550                 555                 560

Ser Trp Ser Asn Thr Lys Ile Ser Val Lys Val Pro Asn Val Ala Gly
                565                 570                 575

Gly Tyr Tyr Asp Leu Ser Val Val Thr Ala Ala Asn Ile Lys Ser Pro
            580                 585                 590

Thr Tyr Lys Glu Phe Glu Val Leu Ser Gly Asn Gln Val Ser Val Arg
        595                 600                 605

Phe Gly Val Asn Asn Ala Thr Thr Ser Pro Gly Thr Asn Leu Tyr Ile
    610                 615                 620

Val Gly Asn Val Asn Glu Leu Gly Asn Trp Asp Ala Asp Lys Ala Ile
625                 630                 635                 640

Gly Pro Met Phe Asn Gln Val Met Tyr Gln Tyr Pro Thr Trp Tyr Tyr
                645                 650                 655

Asp Ile Ser Val Pro Ala Gly Lys Asn Leu Glu Tyr Lys Tyr Ile Lys
            660                 665                 670

Lys Asp Gln Asn Gly Asn Val Val Trp Gln Ser Gly Asn Asn Arg Thr
        675                 680                 685

Tyr Thr Ser Pro Thr Thr Gly Thr Asp Thr Val Met Ile Asn Trp
    690                 695                 700

<210>  15
<211>  711
<212>  PRT
<213>  Bacillus stearothermophilus

<400>  15

Met Arg Arg Trp Leu Ser Leu Val Leu Ser Met Ser Phe Val Phe Ser
1               5                   10                  15

Ala Ile Phe Ile Val Ser Asp Thr Gln Lys Val Thr Val Glu Ala Ala
            20                  25                  30

Gly Asn Leu Asn Lys Val Asn Phe Thr Ser Asp Val Val Tyr Gln Ile
        35                  40                  45

Val Val Asp Arg Phe Val Asp Gly Asn Thr Ser Asn Asn Pro Ser Gly
    50                  55                  60

Ala Leu Phe Ser Ser Gly Cys Thr Asn Leu Arg Lys Tyr Cys Gly Gly

65                  70                  75                  80

Asp Trp Gln Gly Ile Ile Asn Lys Ile Asn Asp Gly Tyr Leu Thr Asp
                85                  90                  95

Met Gly Val Thr Ala Ile Trp Ile Ser Gln Pro Val Glu Asn Val Phe
            100                 105                 110

Ser Val Met Asn Asp Ala Ser Gly Ser Ala Ser Tyr His Gly Tyr Trp
        115                 120                 125

Ala Arg Asp Phe Lys Lys Pro Asn Pro Phe Phe Gly Thr Leu Ser Asp
    130                 135                 140

Phe Gln Arg Leu Val Asp Ala Ala His Ala Lys Gly Ile Lys Val Ile
145                 150                 155                 160

Ile Asp Phe Ala Pro Asn His Thr Ser Pro Ala Ser Glu Thr Asn Pro
                165                 170                 175

Ser Tyr Met Glu Asn Gly Arg Leu Tyr Asp Asn Gly Thr Leu Leu Gly
            180                 185                 190

Gly Tyr Thr Asn Asp Ala Asn Met Tyr Phe His His Asn Gly Gly Thr
        195                 200                 205

Thr Phe Ser Ser Leu Glu Asp Gly Ile Tyr Arg Asn Leu Phe Asp Leu
    210                 215                 220

Ala Asp Leu Asn His Gln Asn Pro Val Ile Asp Arg Tyr Leu Lys Asp
225                 230                 235                 240

Ala Val Lys Met Trp Ile Asp Met Gly Ile Asp Gly Ile Arg Met Asp
                245                 250                 255

Ala Val Lys His Met Pro Phe Gly Trp Gln Lys Ser Leu Met Asp Glu
            260                 265                 270

Ile Asp Asn Tyr Arg Pro Val Phe Thr Phe Gly Glu Trp Phe Leu Ser
        275                 280                 285

Glu Asn Glu Val Asp Ala Asn Asn His Tyr Phe Ala Asn Glu Ser Gly
    290                 295                 300

Met Ser Leu Leu Asp Phe Arg Phe Gly Gln Lys Leu Arg Gln Val Leu
305                 310                 315                 320

Arg Asn Asn Ser Asp Asn Trp Tyr Gly Phe Asn Gln Met Ile Gln Asp
                325                 330                 335

Thr Ala Ser Ala Tyr Asp Glu Val Leu Asp Gln Val Thr Phe Ile Asp
            340                 345                 350

Asn His Asp Met Asp Arg Phe Met Ile Asp Gly Gly Asp Pro Arg Lys
        355                 360                 365

Val Asp Met Ala Leu Ala Val Leu Leu Thr Ser Arg Gly Val Pro Asn
    370                 375                 380

Ile Tyr Tyr Gly Thr Glu Gln Tyr Met Thr Gly Asn Gly Asp Pro Asn
385                 390                 395                 400

Asn Arg Lys Met Met Ser Ser Phe Asn Lys Asn Thr Arg Ala Tyr Gln
                405                 410                 415

Val Ile Gln Lys Leu Ser Ser Leu Arg Arg Asn Asn Pro Ala Leu Ala
            420                 425                 430

Tyr Gly Asp Thr Glu Gln Arg Trp Ile Asn Gly Asp Val Tyr Val Tyr
        435                 440                 445

Glu Arg Gln Phe Gly Lys Asp Val Val Leu Val Ala Val Asn Arg Ser
    450                 455                 460

Ser Ser Ser Asn Tyr Ser Ile Thr Gly Leu Phe Thr Ala Leu Pro Ala
465                 470                 475                 480

Gly Thr Tyr Thr Asp Gln Leu Gly Gly Leu Leu Asp Gly Asn Thr Ile
                485                 490                 495

Gln Val Gly Ser Asn Gly Ser Val Asn Ala Phe Asp Leu Gly Pro Gly
            500                 505                 510

Glu Val Gly Val Trp Ala Tyr Ser Ala Thr Glu Ser Thr Pro Ile Ile
        515                 520                 525

Gly His Val Gly Pro Met Met Gly Gln Val Gly His Gln Val Thr Ile
    530                 535                 540

Asp Gly Glu Gly Phe Gly Thr Asn Thr Gly Thr Val Lys Phe Gly Thr
545                 550                 555                 560

Thr Ala Ala Asn Val Val Ser Trp Ser Asn Asn Gln Ile Val Val Ala
                565                 570                 575

Val Pro Asn Val Ser Pro Gly Lys Tyr Asn Ile Thr Val Gln Ser Ser
            580                 585                 590

Ser Gly Gln Thr Ser Ala Ala Tyr Asp Asn Phe Glu Val Leu Thr Asn
        595                 600                 605

Asp Gln Val Ser Val Arg Phe Val Val Asn Asn Ala Thr Thr Asn Leu
    610                 615                 620

Gly Gln Asn Ile Tyr Ile Val Gly Asn Val Tyr Glu Leu Gly Asn Trp
625                 630                 635                 640

Asp Thr Ser Lys Ala Ile Gly Pro Met Phe Asn Gln Val Val Tyr Ser
                645                 650                 655

Tyr Pro Thr Trp Tyr Ile Asp Val Ser Val Pro Glu Gly Lys Thr Ile
            660                 665                 670

Glu Phe Lys Phe Ile Lys Lys Asp Ser Gln Gly Asn Val Thr Trp Glu
        675                 680                 685

Ser Gly Ser Asn His Val Tyr Thr Thr Pro Thr Asn Thr Thr Gly Lys
    690                 695                 700

Ile Ile Val Asp Trp Gln Asn
705                 710

<210>  16
<211>  655
<212>  PRT
<213>  Klebsiella pneumoniae

<400>  16

Met Lys Arg Asn Arg Phe Phe Asn Thr Ser Ala Ala Ile Ala Ile Ser
1               5                   10                  15

Ile Ala Leu Asn Thr Phe Phe Cys Ser Met Gln Thr Ile Ala Ala Glu
            20                  25                  30

Pro Glu Glu Thr Tyr Leu Asp Phe Arg Lys Glu Thr Ile Tyr Phe Leu
        35                  40                  45

Phe Leu Asp Arg Phe Ser Asp Gly Asp Pro Ser Asn Asn Ala Gly Phe
    50                  55                  60

Asn Ser Ala Thr Tyr Asp Pro Asn Asn Leu Lys Lys Tyr Thr Gly Gly
65                  70                  75                  80

Asp Leu Arg Gly Leu Ile Asn Lys Leu Pro Tyr Leu Lys Ser Leu Gly
                85                  90                  95

Val Thr Ser Ile Trp Ile Thr Pro Pro Ile Asp Asn Val Asn Asn Thr
            100                 105                 110

Asp Ala Ala Gly Asn Thr Gly Tyr His Gly Tyr Trp Gly Arg Asp Tyr
        115                 120                 125

Phe Arg Ile Asp Glu His Phe Gly Asn Leu Asp Asp Phe Lys Glu Leu
    130                 135                 140

Thr Ser Leu Met His Ser Pro Asp Tyr Asn Met Lys Leu Val Leu Asp

145                 150                 155                 160

Tyr Ala Pro Asn His Ser Asn Ala Asn Asp Glu Asn Glu Phe Gly Ala
                165                 170                 175

Leu Tyr Arg Asp Gly Val Phe Ile Thr Asp Tyr Pro Thr Asn Val Ala
            180                 185                 190

Ala Asn Thr Gly Trp Tyr His His Asn Gly Gly Val Thr Asn Trp Asn
        195                 200                 205

Asp Phe Phe Gln Val Lys Asn His Asn Leu Phe Asn Leu Ser Asp Leu
    210                 215                 220

Asn Gln Ser Asn Thr Asp Val Tyr Gln Tyr Leu Leu Asp Gly Ser Lys
225                 230                 235                 240

Phe Trp Ile Asp Ala Gly Val Asp Ala Ile Arg Ile Asp Ala Ile Lys
                245                 250                 255

His Met Asp Lys Ser Phe Ile Gln Lys Trp Thr Ser Asp Ile Tyr Asp
            260                 265                 270

Tyr Ser Lys Ser Ile Gly Arg Glu Gly Phe Phe Phe Phe Gly Glu Trp
        275                 280                 285

Phe Gly Ala Ser Ala Asn Thr Thr Thr Gly Val Asp Gly Asn Ala Ile
    290                 295                 300

Asp Tyr Ala Asn Thr Ser Gly Ser Ala Leu Leu Asp Phe Gly Phe Arg
305                 310                 315                 320

Asp Thr Leu Glu Arg Val Leu Val Gly Arg Ser Gly Asn Thr Met Lys
                325                 330                 335

Thr Leu Asn Ser Tyr Leu Ile Lys Arg Gln Thr Val Phe Thr Ser Asp
            340                 345                 350

Asp Trp Gln Val Val Phe Met Asp Asn His Asp Met Ala Arg Ile Gly
        355                 360                 365

Thr Ala Leu Arg Ser Asn Ala Thr Thr Phe Gly Pro Gly Asn Asn Glu
    370                 375                 380

Thr Gly Gly Ser Gln Ser Glu Ala Phe Ala Gln Lys Arg Ile Asp Leu
385                 390                 395                 400

Gly Leu Val Ala Thr Met Thr Val Arg Gly Ile Pro Ala Ile Tyr Tyr
                405                 410                 415

Gly Thr Glu His Tyr Ala Ala Asn Phe Thr Ser Asn Ser Phe Gly Gln
            420                 425                 430

Val Gly Ser Asp Pro Tyr Asn Arg Glu Lys Met Pro Gly Phe Asp Thr
        435                 440                 445

Glu Ser Glu Ala Phe Ser Ile Ile Lys Thr Leu Gly Asp Leu Arg Lys
    450                 455                 460

Ser Ser Pro Ala Ile Gln Asn Gly Thr Tyr Thr Glu Leu Trp Val Asn
465                 470                 475                 480

Asp Asp Ile Leu Val Phe Glu Arg Arg Ser Gly Asn Asp Ile Val Ile
                485                 490                 495

Val Ala Leu Asn Arg Gly Glu Ala Asn Thr Ile Asn Val Lys Asn Ile
            500                 505                 510

Ala Val Pro Asn Gly Val Tyr Pro Ser Leu Ile Gly Asn Asn Ser Val
        515                 520                 525

Ser Val Ala Asn Lys Arg Thr Thr Leu Thr Leu Met Gln Asn Glu Ala
    530                 535                 540

Val Val Ile Arg Ser Gln Ser Asp Asp Ala Glu Asn Pro Thr Val Gln
545                 550                 555                 560

Ser Ile Asn Phe Thr Cys Asn Asn Gly Tyr Thr Ile Ser Gly Gln Ser
                565                 570                 575

Val Tyr Ile Ile Gly Asn Ile Pro Gln Leu Gly Gly Trp Asp Leu Thr
            580                 585                 590

Lys Ala Val Lys Ile Ser Pro Thr Gln Tyr Pro Gln Trp Ser Ala Ser
        595                 600                 605

Leu Glu Leu Pro Ser Asp Leu Asn Val Glu Trp Lys Cys Val Lys Arg
    610                 615                 620

Asn Glu Thr Asn Pro Thr Ala Asn Val Glu Trp Gln Ser Gly Ala Asn
625                 630                 635                 640

Asn Gln Phe Asn Ser Asn Asp Thr Gln Thr Thr Asn Gly Ser Phe
                645                 650                 655

<210>  17
<211>  686
<212>  PRT
<213>  Bacillus stearothermophilus

<400>  17

Ser Ser Ser Ala Ser Val Lys Gly Asp Val Ile Tyr Gln Ile Ile Ile
1               5                   10                  15

Asp Arg Phe Tyr Asp Gly Asp Thr Thr Asn Asn Asn Pro Ala Lys Ser
            20                  25                  30

Tyr Gly Leu Tyr Asp Pro Thr Lys Ser Lys Trp Lys Met Tyr Trp Gly
        35                  40                  45

Gly Asp Leu Glu Gly Val Arg Gln Lys Leu Pro Tyr Leu Lys Gln Leu
    50                  55                  60

Gly Val Thr Thr Ile Trp Leu Ser Pro Val Leu Asp Asn Leu Asp Thr
65                  70                  75                  80

Leu Ala Gly Thr Asp Asn Thr Gly Tyr His Gly Tyr Trp Thr Arg Asp
                85                  90                  95

Phe Lys Gln Ile Glu Glu His Phe Gly Asn Trp Thr Thr Phe Asp Thr
            100                 105                 110

Leu Val Asn Asp Ala His Gln Asn Gly Ile Lys Val Ile Val Asp Phe
        115                 120                 125

Val Pro Asn His Ser Thr Pro Phe Lys Ala Asn Asp Ser Thr Phe Ala
    130                 135                 140

Glu Gly Gly Ala Leu Tyr Asn Asn Gly Thr Tyr Met Gly Asn Tyr Phe
145                 150                 155                 160

Asp Asp Ala Thr Lys Gly Tyr Phe His His Asn Gly Asp Ile Ser Asn
                165                 170                 175

Trp Asp Asp Arg Tyr Glu Ala Gln Trp Lys Asn Phe Thr Asp Pro Ala
            180                 185                 190

Gly Phe Ser Leu Ala Asp Leu Ser Gln Glu Asn Gly Thr Ile Ala Gln
        195                 200                 205

Tyr Leu Thr Asp Ala Ala Val Gln Leu Val Ala His Gly Ala Asp Gly
    210                 215                 220

Leu Arg Ile Asp Ala Val Lys His Phe Asn Ser Gly Phe Ser Lys Ser
225                 230                 235                 240

Leu Ala Asp Lys Leu Tyr Gln Lys Lys Asp Ile Phe Leu Val Gly Glu
                245                 250                 255

Trp Tyr Gly Asp Asp Pro Gly Thr Ala Asn His Leu Glu Lys Val Arg
            260                 265                 270

Tyr Ala Asn Asn Ser Gly Val Asn Val Leu Asp Phe Asp Leu Asn Thr
        275                 280                 285

Val Ile Arg Asn Val Phe Gly Thr Phe Thr Gln Thr Met Tyr Asp Leu

290                 295                 300

Asn Asn Met Val Asn Gln Thr Gly Asn Glu Tyr Lys Tyr Lys Glu Asn
305                 310                 315                 320

Leu Ile Thr Phe Ile Asp Asn His Asp Met Ser Arg Phe Leu Ser Val
                325                 330                 335

Asn Ser Asn Lys Ala Asn Leu His Gln Ala Leu Ala Phe Ile Leu Thr
            340                 345                 350

Ser Arg Gly Thr Pro Ser Ile Tyr Tyr Gly Thr Glu Gln Tyr Met Ala
        355                 360                 365

Gly Gly Asn Asp Pro Tyr Asn Arg Gly Met Met Pro Ala Phe Asp Thr
    370                 375                 380

Thr Thr Thr Ala Phe Lys Glu Val Ser Thr Leu Ala Gly Leu Arg Arg
385                 390                 395                 400

Asn Asn Ala Ala Ile Gln Tyr Gly Thr Thr Thr Gln Arg Trp Ile Asn
                405                 410                 415

Asn Asp Val Tyr Ile Tyr Glu Arg Lys Phe Phe Asn Asp Val Val Leu
            420                 425                 430

Val Ala Ile Asn Arg Asn Thr Gln Ser Ser Tyr Ser Ile Ser Gly Leu
        435                 440                 445

Gln Thr Ala Leu Pro Asn Gly Ser Tyr Ala Asp Tyr Leu Ser Gly Leu
    450                 455                 460

Leu Gly Gly Asn Gly Ile Ser Val Ser Asn Gly Ser Val Ala Ser Phe
465                 470                 475                 480

Thr Leu Ala Pro Gly Ala Val Ser Val Trp Gln Tyr Ser Thr Ser Ala
                485                 490                 495

Ser Ala Pro Gln Ile Gly Ser Val Ala Pro Asn Met Gly Ile Pro Gly
            500                 505                 510

Asn Val Val Thr Ile Asp Gly Lys Gly Phe Gly Thr Thr Gln Gly Thr
        515                 520                 525

Val Thr Phe Gly Gly Val Thr Ala Thr Val Lys Ser Trp Thr Ser Asn
    530                 535                 540

Arg Ile Glu Val Tyr Val Pro Asn Met Ala Ala Gly Leu Thr Asp Val
545                 550                 555                 560

Lys Val Thr Ala Gly Gly Val Ser Ser Asn Leu Tyr Ser Tyr Asn Ile
                565                 570                 575

Leu Ser Gly Thr Gln Thr Ser Val Val Phe Thr Val Lys Ser Ala Pro
            580                 585                 590

Pro Thr Asn Leu Gly Asp Lys Ile Tyr Leu Thr Gly Asn Ile Pro Glu
        595                 600                 605

Leu Gly Asn Trp Ser Thr Asp Thr Ser Gly Ala Val Asn Asn Ala Gln
    610                 615                 620

Gly Pro Leu Leu Ala Pro Asn Tyr Pro Asp Trp Phe Tyr Val Phe Ser
625                 630                 635                 640

Val Pro Ala Gly Lys Thr Ile Gln Phe Lys Phe Phe Ile Lys Arg Ala
                645                 650                 655

Asp Gly Thr Ile Gln Trp Glu Asn Gly Ser Asn His Val Ala Thr Thr
            660                 665                 670

Pro Thr Gly Ala Thr Gly Asn Ile Thr Val Thr Trp Gln Asn
        675                 680                 685